# EUROPEAN PATENT SPECIFICATION

(11) **EP 1 545 602 B1**
(45) Date of publication and mention of the grant of the patent: **07.05.2008**
(21) Application number: 03784418.0
(22) Date of filing: 12.08.2003
(51) Int. Cl.: A61K 38/17, A61K 39/21, C07K 14/16, C07K 14/705, C07K 16/10, C07K 16/28, C12N 5/06, C12N 15/62, A61P 31/18, A61P 29/00

(54) **PHARMACEUTICAL COMPOSITIONS COMPRISING AN HIV ENVELOPE PROTEIN AND CD4**
PHARMAZEUTISCHE ZUSAMMENSETZUNGEN MIT EINEM HIV-HÜLLPROTEIN UND CD4
COMPOSITIONS PHARMACEUTIQUES RENFERMANT UNE PROTEINE D'ENVELOPPE DU VIH ET CD4

(30) Priority: 13.08.2002 GB 0218817
(43) Date of publication of application: 29.06.2005
(73) Proprietor: FONDAZIONE CENTRO SAN RAFFAELE DEL MONTE TABOR, 20123 Milano (IT)
(72) Inventor: LUSSO, Paolo, Dpt. of Biological & Tech. Research, 20132 Milano (IT); BURASTERO, Samuele, E., Dpt. of Biol.& Tech. Res., 20132 Milano (IT)
(74) Representative: Mallalieu, Catherine Louise
(86) International application number: PCT/IB2003/003665
(87) International publication number: WO 2004/014420

(56) References cited:
- LACASSE R ET AL: "Fusion-competent vaccines: Broad neutralization of primary isolates of HIV" SCIENCE, AMERICAN ASSOCIATION FOR THE ADVANCEMENT OF SCIENCE,, US, vol. 283, no. 5400, 15 January 1999 (1999-01-15), pages 357-362, XP002120812 ISSN: 0036-8075 cited in the application
- NUNBERG JACK H: "Retraction: Fusion-competent vaccines: Broad neutralization of primary isolates of HIV. (Cited as Previews 199900120698.)." SCIENCE (WASHINGTON D C), vol. 296, no. 5570, 10 May 2002 (2002-05-10), page 1025 XP001156542 ISSN: 0036-8075 (ISSN print)
- KANG C Y ET AL: "Immunization with a soluble CD4-gp120 complex preferentially induces neutralizing anti-human immunodeficiency virus type 1 antibodies directed to conformation-dependent epitopes of gp120." JOURNAL OF VIROLOGY. UNITED STATES SEP 1994, vol. 68, no. 9, September 1994 (1994-09), pages 5854-5862, XP009022627 ISSN: 0022-538X cited in the application
- DEVICO ANTHONY ET AL: "Immunogenic properties of HIV gp120-CD4 complexes" JOURNAL OF HUMAN VIROLOGY, vol. 5, no. 1, January 2002 (2002-01), page 71 XP009022616 2002 International Meeting of the Institute of Human Virology;Baltimore, Maryland, USA; September 09-13, 2002 ISSN: 1090-9508
- FOUTS T R ET AL: "Expression and characterization of a single-chain polypeptide analogue of the human immunodeficiency virus type 1 gp120-CD4 receptor complex." JOURNAL OF VIROLOGY. UNITED STATES DEC 2000, vol. 74, no. 24, December 2000 (2000-12), pages 11427-11436, XP001156364 ISSN: 0022-538X
- DEVICO A ET AL: "COVALENTLY CROSSLINKED COMPLEXES OF HUMAN IMMUNODEFICIENCY VIRUS TYPE 1 (HIV-1) GP120 AND CD4 RECEPTOR ELICIT A NEUTRALIZING IMMUNE RESPONSE THAT INCLUDES ANTIBODIES SELECTIVE FOR PRIMARY VIRUS ISOLATES" VIROLOGY, ACADEMIC PRESS,ORLANDO, US, vol. 218, no. 1, 1 April 1996 (1996-04-01), pages 258-263, XP000973669 ISSN: 0042-6822
- CELADA F ET AL: "Antibody Raised Against Soluble CD4-rgp120 Complex Recognizes the CD4 Moiety and Blocks Membrane Fusion without Inhibiting CD4-gp120 Binding" JOURNAL OF EXPERIMENTAL MEDICINE, TOKYO, JP, vol. 172, October 1990 (1990-10), pages 1143-1150, XP002960399 ISSN: 0022-1007
- DEVICO A L ET AL: "MONOCLONAL ANTIBODIES RAISED AGAINST COVALENTLY CROSSLINKED COMPLEXES OF HUMAN IMMUNODEFICIENCY VIRUS TYPE 1 GP120 AND CD4 RECEPTOR IDENTIFYA NOVEL COMPLEX-DEPENDENT EPITOPE ON GP120" VIROLOGY, ACADEMIC PRESS,ORLANDO, US, vol. 211, no. 2, 20 August 1995 (1995-08-20), pages 583-588, XP000578383 ISSN: 0042-6822 cited in the application
- MARTIN LOÏC ET AL: "Rational design of a CD4 mimic that inhibits HIV-1 entry and exposes cryptic neutralization epitopes." NATURE BIOTECHNOLOGY. UNITED STATES JAN 2003, vol. 21, no. 1, January 2003 (2003-01), pages 71-76, XP001156366 ISSN: 1087-0156
- PAUL N L ET AL: "Expression of HIV-1 envelope glycoproteins by Semliki Forest virus vectors." AIDS RESEARCH AND HUMAN RETROVIRUSES. UNITED STATES OCT 1993, vol. 9, no. 10, October 1993 (1993-10), pages 963-970, XP009022614 ISSN: 0889-2229
- SCHUBERT M ET AL: "INSERTION OF THE HUMAN IMMUNODEFICIENCY VIRUS CD4 RECEPTOR INTO THE ENVELOPE OF VESICULAR STOMATIS VIRUS PARTICLES" JOURNAL OF VIROLOGY, NEW YORK, US, US, vol. 66, no. 3, 1 March 1992 (1992-03-01), pages 1579-1589, XP000644669 ISSN: 0022-538X

## Description

### Field of the Invention

The present invention relates to pharmaceutical compositions and fixed cells and to their use in the prevention and treatment of HIV infection and disorders related to the immune system.

### Background of the Invention

The human retrovirus HIV (human immunodeficiency virus) has been demonstrated to be the causative agent of acquired immunodeficiency syndrome (AIDS), a disease for which there is currently no cure. Even though more than 20 years have elapsed since the first description of AIDS, no effective "preventive" vaccine is yet available against infection with HIV. The rapid rise in seropositivity among individuals in high risk categories, the virulence of the disease, and its growing world-wide distribution, underscore an overwhelming and immediate need for a vaccine capable of inducing complete protective immunity in non-infected individuals.

Induction of a *bona fide* protective immunity could be achieved by a vaccine that elicits the production of viral antigen-specific antibodies targeted at the external coat of the virus (envelope) which are capable of neutralizing the early events of the viral life cycle (i.e., attachment to the cellular membrane, fusion and entry). However, all the experience accrued in this field over a period of more than a decade has clearly demonstrated that the development of such a vaccine represents an extraordinary challenge.

HIV encodes an envelope glycoprotein precursor, gp160, which is proteolytically cleaved into the exterior (gp120) and transmembrane (gp41) glycoproteins (Allan et al., 1985; Kowalski et al., 1987). The gp120 glycoprotein remain associated with the mature envelope glycoprotein complex through a non-covalent interaction with the gp41 ectodomain (Veronese et al., 1985).

Current strategies for developing vaccines against infection by HIV have focused on eliciting antibodies against the external viral envelope glycoprotein gp120 or its cell surface receptor CD4. However, attempts to generate anti-HIV-1 vaccines based on purified, soluble forms of gp120 have been unsuccessful. Reasons for this failure include, firstly, the extraordinary propensity of gp120 to mutate its primary and secondary structure, thereby making the virus a continuously "moving target", and, secondly, the concealment of critical neutralization epitopes expressed on the envelope surface by locating them inside deep surface cavities or by presenting them in cryptic form (Kwong et al., 1998).

New hope was raised by the observation that some of the cryptic conserved neutralization epitope on the gp120 glycoprotein, specifically those involved in coreceptor binding (Thali et al., 1993), as well as others, including the principal neutralization domain or "V3 loop" (Sattentau and Moore, 1991; Mbah et al., 2001; Lusso and Burastero, unpublished data), become exposed and antibody-accessible upon binding of gp120 with the CD4 receptor, leaving to virus-cell membrane fusion. Several groups have explored the possibility of immunizing with a complex between gp120 and soluble CD4 (sCD4) in an attempt to present critical neo-epitopes to the immune system, which are unravelled by the receptor/envelope interaction (Celada et al., 1990; Kang et al., 1994; DeVico et al., 1995). More recently, one group has produced a single synthetic chimeric molecule encompassing both gp 120 and a truncated CD4 molecule linked via a flexible linker that allows for intramolecular gp120-CD4 complex formation (Fouts et al., 2000).

The present invention identifies a problem associated with the aforementioned approaches. In its native form, the HIV envelope comprises an oligomeric complex of gp120 and gp41 subunits. The oligomeric complex is most likely a trimer comprising three gp120 and three gp41 subunits. This has important implications for the accessibility and conformation of several neutralisation epitopes (Yang et al., 2001): some of them are well exposed on the monomer but are not available to antibody-mediated neutralisation since they are hidden on the trimer (Sattentau et al., 1995; Nyambi et al., 1998). Converesely, other epitopes may only be presented in the native oligomeric form. Thus, the use of soluble gp120-sCD4 complexes fails to present many of the most important neo-epitopes expressed by the HIV envelope protein during viral infection.

Importantly the associations between the six components of the fusion-competent envelope complex are relatively weak, making the complex unstable. Moreover, since the native complex falls apart before it can be purified, its use as an immunogen has been severely limited.

The concept of presenting to the immune system neo-epitopes of native HIV envelope that become visible at a transitional stage induced by interaction of the viral envelope with the receptor complex (CD4 and coreceptor) has been developed by LaCasse et al. (Science 1999), who used whole cells expressing the HIV envelope "frozen" (by chemical fixation) at random intermediate stages of fusion with cells expressing the receptor complex. However, a retraction of the published results revealed that only non-specific neutralisation due to an unknown cytotoxic effect of the complex sera was observed (Science 2002 May 10;296(5570):1025).

CD4 receptor plays a key role in eliciting an immune response. CD4+ T cells recognise antigen which has been processed and is presented in association with a self class II MHC molecule. In initiating the immune response, this interaction takes place in the lymphoid tissue, and involves T cell interaction with professional antigen presenting cells which take up, process and present the relevant antigen. Previous work has shown that treatment with antibodies specific for CD4 may be valuable in preventing a range of both experimentally-induced and spontaneously-occurring autoimmune diseases.

There remains a need to develop neutralising antigens that present to the immune system epitopes that are exposed during in-vivo attachment of HIV envelope protein to the receptor of target cells and to develop neutralising antigens that present to the immune system epitopes that are exposed during during T cell interactions with antigen presenting cells. The present invention overcomes this problem.

### Summary of the invention

The invention relates to novel strategies for treatment and immunization against HIV and T-cell mediated diseases. In particular, the invention provides for both 'in-vivo' and 'ex-vivo' generation of HIV envelope-CD4 complexes.

Thus, according to one aspect of the present invention there is provided a pharmaceutical composition comprising (i) a vector comprising a polynucleotide encoding a gp160 HIV envelope protein and (ii) a vector comprising a polynucleotide encoding a CD4 receptor wherein the vectors are independently selected from the group comprised of a lentivirus, a murine leukemia virus, a herpes virus, a mouse mammary tumor virus a rous sarcoma virus, a fujinami sarcoma virus, an FBR murine osteosarcoma virus, a moloney murine sarcoma virus, an avian myclocytomatosis virus-29 and an avian erythroblastosis virus.

Preferably, said HIV envelope protein and said CD4 receptor encoded by (i) and (ii) are expressed as separate proteins. That is, they are not linked or fused together.

The polynucleotides are encoded on vectors. Preferably, the vectors are selected from the group comprising Murine Leukemia Virus, Lentiretroviruses and Herpesviruses.

The polynucleotide of (ii) may be encoded on the same or on a different vector to the polynucleotide of (i).

Thus in one aspect of the invention there is provided a pharmaceutical composition as defined above wherein (i) are (ii) are encoded on separate vectors.

In another aspect of the invention there is provided a pharmaceutical composition as defined above wherein (i) and (ii) are encoded on the same vector.

Alternatively, in another aspect of the invention, there is provided a pharmaceutical composition comprising (i) a polynucleotide encoding a gp160 HIV envelope protein and (ii) a CD4 receptor polypeptide.

The pharmaceutical composition preferably comprises several polynucleotides encoding HIV envelope proteins, each of which may originate from different HIV subtypes. These polynucleotides may exist on the same or on separate vectors.

In one embodiment the HIV envelope protein is expressed as part of a fusion protein and/or the CD4 receptor is expressed as part of a fusion protein.

In another embodiment, the CD4 receptor employed in the invention is in the form of a fusion protein.

Preferably the pharmaceutical composition according to the invention comprises a pharmaceutically acceptable diluent or excipient.

In a further aspect of the present invention there is provided a pharmaceutical composition of the present invention for use in the treatment of HIV.

In another aspect of the present invention there is provided a pharmaceutical composition of the present invention in the form of a vaccine.

In a further aspect of the present invention there is provided the use of a pharmaceutical composition of the present invention for the preparation of anti-HIV monoclonal antibodies.

Preferably the administration of the pharmaceutical composition is in proximity of a superficial lymph-node station.

In another aspect of the present invention there is provided the use of a pharmaceutical composition of the present invention for the preparation of a medicament for use in generating an immune response against HIV infection.

In another aspect of the present invention there is provided the use of a pharmaceutical composition of the present invention for the preparation of a medicament for use in preventing or treating HIV infection.

In a further aspect of the present invention there is provided a delivery system for (i) a polynucleotide encoding an HIV envelope protein and (ii) a polynucleotide encoding a CD4 receptor.

Preferably, said HIV envelope protein and said CD4 receptor encoded by (i) and (ii) are expressed as separate proteins. That is, they are not linked or fused together.

In a further aspect of the present invention there is provided a delivery system for (i) a polynucleotide encoding an HIV envelope protein and (ii) a CD4 receptor.

In another aspect of the present invention there is provided the use of a delivery system of the present invention for treating or preventing HIV infection.

The invention further relates to HIV envelope-CD4 complexes provided in the form of a fixed cell comprising a gp160 HIV envelope protein complexed with a CD4 receptor.

Thus according to a further aspect of the present invention there is provided a fixed cell wherein said cell expresses a gp160 HIV envelope protein and further wherein said envelope protein is complexed with a CD4 receptor.

In one embodiment, the fixed cell is derived from a T-cell. In another embodiment, the fixed cell is derived from an NIH-3T3 cell.

In another embodiment the fixed cell is fixed with glutaraldehyde.

In a further aspect of the present invention, there is provided a fixed cell of the invention for use in medicine, in particular for use in treating or preventing HIV infection and diseases or conditions of, or related to the immune system, including T-cell mediated disorders and inflammation. Preferably the T-cell mediated disorder is a CD4+ T-cell mediated disorder.

In another aspect of the present invention there is provided the use of a fixed cell of the invention for the preparation of a medicament for use in generating an immune response against HIV infection.

In another aspect of the present invention there is provided the use of a fixed cell of the invention for the preparation of a medicament for use in treating or preventing HIV infection

In another aspect of the present invention there is provided the use of a fixed cell of the present invention for the preparation of a medicament for use in treating a disease or condition of, or related to, the immune system, including T-cell mediated disorders and inflammation. Preferably the T-cell mediated disorder is a CD4+ T-cell mediated disorder.

In another aspect of the present invention there is provided a vaccine comprising the fixed cell of the invention.

In another aspect of the present invention there is provided an antibody immunospecific for the fixed cell of the invention. In other words, an antibody which may be generated against the fixed cell of the present invention.

In another aspect of the present invention there is provided an antibody capable of binding to the fixed cell of the present invention.

In a preferred embodiment the antibody is immunospecific for CD4 bound to gp120. In other words, an antibody which may be generated against CD4-gp120 complex. Preferably the antibody has greater specificity for the CD4-gp120 complex than either gp 120 or CD4 alone.

In another aspect of the present invention, there is provided an antibody of the present invention for use in medicine.

In another aspect of the present invention there is provided the use of an antibody of the present invention for the preparation of a medicament for treating or preventing HIV infection.

In another aspect of the present invention there is provided a hybridoma cell line having the identifying characteristics of the cell line deposited with the Advanced Biotechnology Center Inter Lab Cell Line Collection (CBA ICLB) wherein said hybridoma produces the antibody DB-81.

In another embodiment, there is provided a hybridoma cell line deposited with the Advanced Biotechnology Center Inter Lab Cell Line Collection (CBA ICLB) wherein said hybridoma produces the antibody DB-81.

In another aspect of the present invention there is provided the antibody DB-81.

In another aspect of the present invention, there is provided an antibody that specifically binds to substantially the same antigen epitopes as DB-81. In another aspect of the present invention, there is provided an antibody that specifically binds the same idiotope as antibody DB-81.

In another aspect of the present invention there is provided the use of an antibody of the present invention for the preparation of a medicament for use in treating a disease or condition of, or related to, the immune system, including T-cell mediated disorders and inflammation. Preferably the T-cell mediated disorder is a CD4+ T-cell mediated disorder.

In a further aspect of the invention there is provided a method of generating a fixed cell according to the invention comprising
(i) expressing an HIV- envelope protein encoded by gp160 on the surface of living cells;
(ii) complexing the envelope protein of (i) with a CD4 receptor; and
(iii) fixing the CD4 coated cells.

In a preferred embodiment the HIV envelope protein is expressed on the surface of the living cell by transducing the cell with a vector encoding the HIV envelope protein. In one embodiment, the vector is a vaccinia vector.

In another embodiment the CD4 receptor is expressed from the living cell.

In another embodiment the living cell expresses the HIV envelope protein and the CD4 receptor from a single vector.

In another aspect of the present invention there is provided a pharmaceutical composition comprising at least one fixed cell of the invention and a pharmaceutically acceptable diluent or excipient.

In another aspect of the present invention there is provided a pharmaceutical composition comprising a population of fixed cells of the present invention, wherein within the population more than one HIV envelope serotype is expressed, and a pharmaceutically acceptable diluent or excipient.

In one embodiment the CD4 receptor as defined above in relation to certain aspects and embodiments of the invention includes soluble CD4 receptor.

Preferably, the CD4 receptor is the human CD4 receptor.

By "CD4 receptor" we include CD4 surrogate molecules including but not limited to M33 mini-protein.

The HIV envelope protein used in the present invention may be HIV-1 or HIV-2 envelope protein and is preferably HIV-1 envelope protein.

In one embodiment the HIV envelope protein is the envelope protein of a primary HIV-1 subtype that utilises CCR5 coenzyme. CCR5-using isolates are the typical viral subtypes transmitted in vivo both in children (Scarlatti et al., 1997) and in adults. In another embodiment the HIV envelope protein is the envelope protein of a primary HIV-1 subtype that utilises CXCR4 coenzyme.

The polynucleotide encoding the HIV envelope protein of the present invention encodes HIV gp 160.

### Detailed description

Various preferred features and embodiments of the present invention will now be described by way of non-limiting examples.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992 and periodic supplements, Current Protocols in Immunology, John Wiley & Sons, New York, NY.

The present invention provides a novel approach of presenting to the immune system neo-epitopes that are only transiently exposed during the process of viral envelope-CD4 interaction that leads to virus-cell fusion during HIV infection. According to one aspect of the present invention, this may be achieved by the ex-vivo or in-vivo expression of HIV envelope protein and CD4 receptor, preferably using viral vectors.

Preferably, the HIV envelope and CD4 receptor are expressed in different cells. Alternatively, the CD4 receptor may be administered as a soluble protein. Expression of HIV envelope protein on the surface of transduced cells together with local release of sCD4 receptor may generate an immunogenic complex.

The immunogenic complex of the present invention may also be in the form of a fixed cell wherein said cell expresses an HIV envelope protein and further wherein envelope protein is complexed with CD4 receptor.

A number of advantages of the present invention are that:
- the oligomeric conformation of the viral envelope is maintained and is expressed on whole cells
- the viral envelope-CD4 complex can be generated *in vivo* improving the efficacy and specificity of the response
- the problem associated with the intrinsic instability associated with HIV envelope protein may be overcome
- vaccines comprising vectors are practical and easy to administer

Other advantages are discussed and are made apparent in the following commentary:

As used herein the terms "comprising" and "having" relate to not only to anything consisting of a specified feature or characteristic, but also anything including that feature or characteristic as well as one or more additional features or characteristics.

### HIV envelope protein

Reference herein to HIV envelope protein includes variants, derivatives and homologues thereof, and polynucleotides encoding the same.

The native HIV envelope comprises a oligomeric structure composed of gp120 subunits and gp41 subunits. The receptor-binding (CD4 and coreceptor) sites are located in the gp120 moieties, and the fusion peptides are located in the gp41 components (Chan et al., 1997; Kwong et al., 1998; Kwong et al. (2000) J. Virol. 74:1961). The associations between the components of the fusion-competent complex are maintained via non-covalent interactions between gp120 and gp41 and between the gp41 subunits.

HIV cell entry is a multistep process that begins when gp120 engages CD4 on the cell surface. The two most common coreceptors used by HIV-1 are the chemokine receptors CCR5 and CXCR4 (Berger et al., 1997, Berger et al., 1998). Coreceptor binding by gp120 triggers a change in the conformation of gp41 that exposes its hydrophobic fusion domain and permits insertion of the N-terminal peptide of gp41 into the cytoplasmic membrane of target cells (Weissenhorn et al., 1997; Chan et al., 1998; Chan et al., 1997). Further structural changes in this fusogenic form of gp41 then draw the virus and the cell membranes into close proximity to complete the fusion process. CXCR4 is the receptor for the chemokine SDF-1 (Bleul et al., 1996), whereas CCR5 serves as a receptor for the chemokines MIP-1 α and β as well as RANTES (Murphy, 1996).

The HIV envelope protein used in the present invention preferably comprises oligomeric gp120-gp41 complex. The oligomeric gp120-gp41 complex may be a trimeric complex comrising three gp120 subunits and three gp41 subunits.

The HIV envelope protein used in the present invention may be from HIV-1 or HIV-2 and is preferably HIV-1 envelope protein.

The polynucleotide employed by the invention to encode the HIV envelope protein comprises the gp160 gene. The protein encoded by gp160 is proteolytically cleaved into the gp120 and gp41 glycoproteins intracellularly.

In another embodiment, the HIV envelope protein used in the present invention is a gp140 trimer comprising uncleaved ectodoamins of gp 120 and gp41. gp140 trimers may be stabilised by, for example, fusion with a C-terminal trimeric motif (Yang et al., 2002) or the addition of an intersubunit disulphide bond (Earl et al., 2002).

Preferably, the HIV envelope proteins are derived from a primary CCR5-dependent HIV-1 isolate, i.e., the predominate subtype that is transmitted from person-to-person *in vivo*.

HIV envelope proteins originating from different HIV subtypes may be used in the present invention. It will be understood by a skilled person that an effective vaccine to HIV may provide protection against several serotypes. Thus, a pharmaceutical composition or vaccine according to the invention may include polynucleotides that encode for more than one HIV envelope protein from different HIV serotypes. A pharmaceutical composition or vaccine of the invention may also include a fixed cell according to the invention that comprises CD4 complexed to HIV envelope proteins from different HIV serotypes. Alternatively, a pharmaceutical composition or vaccine of the invention may include more than one fixed cell, each fixed cell comprising CD4 complexed to HIV envelope protein from a different HIV serotype.

Of the two major HIV types, HIV-1 and HIV-2, HIV-1 is the most virulent and is the predominant species around the world. To date, two major groups of HIV-1 exist, "M" and "O". The virus that causes the great majority of HIV-1 infections are in the M group. The O group isolates are genetically quite distant from the M group. HIV-1 subtypes of the M group include subtypes A-J. Subtype A is the most genetically diverged subtype. Subtype A is mostly prevalent in Central Africa, but has also been found in other parts of the world, including Europe, Russia, East Asia and America. Subtype B represents the vast majority of subtypes circulating in the western world. Subtype E has also entered the American continent, including the USA, and subtype E variants have been found in Central Africa and Asia. Subtype C seems to have its major centre in East and South Africa, but has been reported to occur in many other parts of the world, including Europe, Russia, China, India, and Brazil.

Examples of HIV envelope protein sequences are readily available from commercial and institutional HIV sequence databases, such as GENBANK, or as published compilations.

### CD4 Receptor

Reference herein to CD4 receptor include variants, derivatives, and homologues thereof, and polynucleotides encoding the same.

The CD4 receptor belongs to the immunoglobulin superfamily (Maddon et al., 1985) with four extracellular domains (D1-D4). The gp120 binding site is contained in the N-terminal D1 domain. In the CD4-gp120 complex, the CD4 surface binding to gp120 is centered on the CDR2-like loop, a protruding β-hairpin with its C" β-strand forming an antiparallel β-sheet with the gp120 β15 strand. The major binding epitope for gp 120 appears to be between residues 40-60 (Peterson and Seed, 1988; Arthos et al., 1989), which is homologous to the CDR2 site of the immunoglobulin light chain. Residues 43 and 59 are particularly important for gp120 recognition with the Phe43 side chain occupying the entrance of the gp120 cavity, and the Arg59 side chain forming a double H-bond with the gp120 Asp368 side-chain (Kwong et al., 1998; Kwong et al., (2000) Structure Fold. Des. 8:1329). Another domain at residues 80-100 resembling CDR3 of Ig has also been considered to be important either for gp120 binding or for events leading up to fusion after initial binding (Camerini and Seed, 1990; Ohki et al., 1990; Lifson et al., 1991; Truneh ey al., 1991).

In one embodiment, the CD4 receptor used in the invention is soluble human CD4 receptor. Soluble CD4 receptors are well known in the art.

In another embodiment, the CD4 receptor used in the invention is human CD4 receptor.

A truncated form of soluble CD4 which is smaller than the wild-type CD4 and well suited for expression in living cells, may be used in the present invention. An example of such a molecule is described by Berger (1988) Proc Natl Acad Sci U S A 85:2357. Alternatively, other molecular forms, modified mutants or CD4-mimicking molecules based or not based on CD4 sequences may be used.

A surrogate CD4 molecule may be used in the present invention. Surrogate CD4 molecules may exhibit all major functional characteristics of native human CD4, while offering important advantages such as small size and high stability. Surrogate molecules employed by the invention may include CD4 miniproteins such as those described by Vita (Vita et al., 1999) and the M33 mini-protein described by Vita et al., 2003, may be used in the present invention.

CD4 or CD4-mimetic agents can be administered as pre-formed proteins, produced by chemical synthesis or recombinant technologies, or alternatively, they can be engineered into plasmid DNA or viral vectors, or other forms of gene delivery systems. Such delivery systems may be used to drive expression *in-vivo* at the site of delivery.

### Polynucleotides

Polynucleotides used in the invention may comprise DNA or RNA. They may be single-stranded or double-stranded. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides used in the invention to reflect the codon usage of any particular host organism in which the polypeptides are to be expressed. The polynucleotides may be modified by any method available in the art. Such modifications may be carried out in order to enhance the *in vivo* activity or life span of polynucleotides of the invention.

The polynucleotides used in the invention may encode fusion proteins for example to aid in cellular secretion of the expressed polypeptides. Where, for example, two polypeptides of the invention, e.g., HIV envelope protein and CD4 receptor, are desired to be expressed from the same cell, it may be desirable to add targeting sequences to target the proteins to particular cell compartments or to secretory pathways. Such targeting sequences have been extensively characterised in the art.

Polynucleotides such as DNA polynucleotides may be produced recombinantly, synthetically, or by any means available to those of skill in the art. They may also be cloned by standard techniques.

Longer polynucleotides will generally be produced using recombinant means, for example using PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

### Protein

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. The terms subunit and domain may also refer to polypeptides and peptides having biological function.

### Variants, Derivatives, Homologues and Fragments

In addition to the specific proteins and nucleotides mentioned herein, the present invention also encompasses the use of variants, derivatives, homologues and fragments thereof.

In the context of the present invention, a variant of any given sequence is a sequence in which the specific sequence of residues (whether amino acid or nucleic acid residues) has been modified in such a manner that the polypeptide or polynucleotide in question retains at least one of its endogenous functions. A variant sequence can be modified by addition, deletion, substitution modification replacement and/or variation of at least one residue present in the naturally-occurring protein.

The term "derivative" as used herein, in relation to proteins or polypeptides of the present invention includes any substitution of, variation of, modification of, replacement of, deletion of and/or addition of one (or more) amino acid residues from or to the sequence providing that the resultant protein or polypeptide retains at least one of its endogenous functions.

Typically, amino acid substitutions may be made, for example from 1, 2 or 3 to 10 or 20 substitutions provided that the modified sequence retains the required activity or ability. Amino acid substitutions may include the use of non-naturally occurring analogues.

Proteins of use in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the transport or modulation function is retained. For example, negatively charged amino acids include aspartic acid and glutamic acid; positively charged amino acids include lysine and arginine, and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

Polynucleotide variants will preferably comprise codon optimised sequences. Codon optimisation is known in the art as a method of enhancing RNA stability and therefor gene expression. The redundancy of the genetic code means that several different codons may encode the same amino-acid. For example, Leucine, Arginine and Serine are each encoded by six different codons. Different organisms show preferences in their use of the different codons. Viruses such as HIV, for instance, use a large number of rare codons. By changing a nucleotide sequence such that rare codons are replaced by the corresponding commonly used mammalian codons, increased expression of the sequences in mammalian target cells can be achieved. Codon usage tables are known in the art for mammalian cells, as well as for a variety of other organisms. Preferably, at least part of the sequence is codon optimised. Even more preferably, the sequence is codon optimised in its entirety.

### Vectors

As it is well known in the art, a vector is a tool that allows or facilitates the transfer of an entity from one environment to another. In accordance with the present invention, and by way of example, some vectors used in recombinant DNA techniques allow entities, such as a segment of DNA (such as a heterologous DNA segment, such as a heterologous cDNA segment), to be transferred into a host and/or a target cell for the purpose of replicating the vectors comprising the nucleotide sequences used in the invention and/or expressing the proteins used in the invention. Examples of vectors used in recombinant DNA techniques include but are not limited to plasmids, chromosomes, artificial chromosomes or viruses.

Polynucleotides used in the invention are preferably incorporated into a vector. Preferably, a polynucleotide in a vector for use in the invention is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under conditions compatible with the control sequences.

The control sequences may be modified, for example by the addition of further transcriptional regulatory elements to make the level of transcription directed by the control sequences more responsive to transcriptional modulators.

Vectors may be transformed or transfected into a suitable host to provide for expression of CD4 for use in present the invention. This process may comprise culturing a host cell transformed with an expression vector as described above under conditions to provide for expression by the vector of a coding sequence encoding the protein, and optionally recovering the expressed protein.

The vectors used in the present invention may be for example, plasmid or virus vectors provided with an origin of replication, optionally a promoter for the expression of a polynucleotide and optionally a regulator of the promoter. The vectors may contain one or more selectable marker genes, and/or a traceable marker such as GFP. Vectors may be used, for example, to transfect or transform a host cell.

Control sequences operably linked to sequences encoding proteins for use in the invention include promoters/enhancers and other expression regulation signals. These control sequences may be selected to be compatible with the host cell for which the expression vector is designed to be used in. The term "promoter" is well-known in the art and encompasses nucleic acid regions ranging in size and complexity from minimal promoters to promoters including upstream elements and enhancers.

The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of a-actin, b-actin, tubulin) or, alternatively, a tissue-specific manner (such as promoters of the genes for pyruvate kinase). Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

The vector used in the present invention may be a retrovirus based vector which has been genetically engineered so that it can not replicate and produce progeny infectious virus particles once the virus has entered the target cell. There are many retroviruses that are widely used for delivery of genes both in tissue culture conditions and in living organisms. Examples include and are not limited to murine leukemia virus (MLV), human immunodeficiency virus (HIV-1), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other *retroviridiae* including lentiviruses. A detailed list of retroviruses may be found in Coffin et al., 1997, "retroviruses", Cold Spring Harbour Laboratory Press Eds: JM Coffin, SM Hughes, HE Varmus pp 758-763.

Preferably, the vectors used in the present invention are selected from the group comprising Murine Leukemia Virus, Lentiretroviruses, Herpesviruses, Adenovirus, Adeno-associated virus and Semliki Forest Virus.

The adenovirus is a double-stranded, linear DNA virus that does not go through an RNA intermediate. There are over 50 different human serotypes of adenovirus divided into 6 subgroups based on the genetic sequence homology. The natural target of adenovirus is the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Viral gene expression can be divided into early (E) and late (L) phases. The late phase is defined by the onset of viral DNA replication. Adenovirus structural proteins are generally synthesised during the late phase. Following adenovirus infection, host cellular mRNA and protein synthesis is inhibited in cells infected with most serotypes. The adenovirus lytic cycle with adenovirus 2 and adenovirus 5 is very efficient and results in approximately 10, 000 virions per infected cell along with the synthesis of excess viral protein and DNA that is not incorporated into the virion. Early adenovirus transcription is a complicated sequence of interrelated biochemical events but it entails essentially the synthesis of viral RNAs prior to the onset of DNA replication.

The organisation of the adenovirus genome is similar in all of the adenovirus groups and specific functions are generally positioned at identical locations for each serotype studied. Early cytoplasmic messenger RNAs are complementary to four defined, noncontiguous regions on the viral DNA. These regions are designated E1-E4. The early transcripts have been classified into an array of intermediate early (E1a), delayed early (E1b, E2a, E2b, E3 and E4), and intermediate regions.

The early genes are expressed about 6-8 hours after infection and are driven from 7 promoters in gene blocks E1-4.

Adenoviruses may be converted for use as vectors for gene transfer by deleting the E1 gene, which is important for the induction of the E2, E3 and E4 promoters. The E1-replication defective virus may be propagated in a cell line that provides the E1 polypeptides in trans, such as the human embryonic kidney cell line 293. A therapeutic gene or genes can be inserted by recombination in place of the E1 gene. Expression of the gene is driven from either the E1 promoter or a heterologous promoter.

Even more attenuated adenoviral vectors have been developed by deleting some or all of the E4 open reading frames (ORFs). However, certain second generation vectors appear not to give longer-term gene expression, even though the DNA seems to be maintained. Thus, it appears that the function of one or more of the E4 ORFs may be to enhance gene expression from at least certain viral promoters carried by the virus.

An alternative approach to making a more defective virus has been to "gut" the virus completely maintaining only the terminal repeats required for viral replication. The "gutted" or "gutless" viruses can be grown to high titres with a first generation helper virus in the 293 cell line but it has been difficult to separate the "gutted" vector from the helper virus.

The adenovirus provides advantages as a vector for gene transfer for the following reasons:

It is a double stranded DNA nonenveloped virus that is capable of *in vivo* and *in vitro* transduction of a broad range of cell types of human and non-human origin. These cells include respiratory airway epithelial cells, hepatocytes, muscle cells, cardiac myocytes, synoviocytes, primary mammary epithelial cells and post-mitotically terminally differentiated cells such as neurons.

Adenoviral vectors are also capable of transducing non dividing cells. This is very important for diseases, such as cystic fibrosis, in which the affected cells in the lung epithelium, have a slow turnover rate. In fact, several trials are underway utilising adenovirus-mediated transfer of cystic fibrosis transporter (CFTR) into the lungs of afflicted adult cystic fibrosis patients.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, it functions episomally (independently from the host genome) as a linear genome in the host nucleus. Hence the use of recombinant adenovirus alleviates the problems associated with random integration into the host genome.

Pox viral vectors may be used in accordance with aspects of the present invention, as large fragments of DNA are easily cloned into its genome and recombinant attenuated vaccinia variants have been described (Meyer et al., 1991; Smith and Moss, 1983).

Herpes viral vectors may be derived from, for example, HSV1 or HSV2 strains, or derivatives thereof, preferably HSV1. Derivatives include inter-type recombinants containing DNA from HSV1 and HSV2 strains. Derivatives preferably have at least 70% sequence homology to either the HSV1 or HSV2 genomes, more preferably at least 90%, even more preferably 95%.

The use of HSV (Herpes Simplex Virus) strains in therapeutic procedures will require the strains to be attenuated so that they cannot establish a lytic cycle. In particular, if HSV vectors are to be used for therapy in humans the polynucleotide should preferably be inserted into an essential gene. This is because if a vector virus encounters a wild-type virus transfer of a heterologous gene to the wild-type virus could occur by recombination. However as long as the polynucleotide is inserted into an essential gene this recombinational transfer would also delete the essential gene in the recipient virus and prevent 'escape' of the heterologous gene into the replication competent wild-type virus population.

Attenuated HSV strains, here given as examples only, that may be used in the present invention include strains that have mutations in either ICP34.5 or ICP27, for example strain 1716 (MacLean et al., 1991), strains R3616 and R4009 (Chou and Roizman, 1992), and R930 (Chou et al., 1994) all of which have mutations in ICP34.5, and d27-1 (Rice and Knipe, 1990) which has a deletion in ICP27. Alternatively strains deleted for ICP4, ICP0, ICP22, ICP6, ICP47, *vhs* or gH, with an inactivating mutation in VMW65, or with any combination of the above may also be used.

The terminology used in describing the various HSV genes is as found in Coffin and Latchman, 1996. Herpes simplex virus-based vectors. In: Latchman DS (ed). Genetic manipulation of the nervous system. Academic Press: London, pp 99-114.

The lentivirus group includes both "primate" and "non-primate" lentiviruses. Examples of primate lentiviruses include human immunodeficiency virus (HIV-1), the causative agent of human auto-immunodeficiency syndrome (AIDS), and simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

A distinction between the lentivirus family and other types of retroviruses is that lentiviruses have the capability to infect both dividing and non-dividing cells. In contrast, other retroviruses - such as MLV - are unable to infect non-dividing cells such as those that make up, for example, muscle, brain, lung and liver tissue. Thus, lentiviral vectors may advantageously be used in the present invention since lentiviruses are capable of infecting a wide range of non-dividing cells, by contrast to certain other retroviruses that require cell division for stable integration.

The vectors used in the present invention may be specifically designed to carry bi-cistronic polynucleotide sequences coding for proteins used in the present invention.

### Delivery Systems

The invention further provides a delivery system for (i) a polynucleotide encoding HIV envelope protein, and (ii) a CD4 receptor or a polynucleotide encoding the same. (i) and (ii) may be delivered simultaneously or separately.

The delivery system of the present invention may be a viral or non-viral delivery system. Non-viral delivery mechanisms include but are not limited to lipid mediated transfection, liposomes, immunoliposomes, lipofectin, cationic facial amphiphiles (CFAs) and combinations thereof.

The polynucleotides may be delivered to the target cell population by any suitable Gene Delivery Vehicle, GDV. This includes but is not restricted to, DNA, formulated in lipid or protein complexes or administered as naked DNA via injection or biolistic delivery, viruses such as retroviruses, adenoviruses, poxvirus, lentiviruses, herpes viruses, vaccinia viruses, adeno associated viruses, murine leukemia viruses, semliki forest viruses and baculoviral viruses. Alternatively, the polynucleotide are delivered by cells such as monocytes, macrophages, lymphocytes or hematopoietic stem cells. In particular a cell-dependent delivery system is used. In this system the polynucleotides encoding the CD4 receptor and the HIV envelope protein are introduced into one or more cells *ex vivo* and then introduced into the patient.

The agents of the present invention may be administered alone but will generally be administered as a pharmaceutical composition.

### Fixed Cells

As used herein, the term "fixed cell" refers to a cell that has undergone the process of fixation. The term "fixation" refers to a process that stabilises cell components and halts cell processes with a minimum alteration from the living state. Methods of cell fixation are well known in the art.

A fixed cell of the present invention may be derived from an animal cell that is cap able of expressing HIV envelop e protein on its surface.

Preferably, the animal cell is a cell of the immune system, e.g., a T-cell. Preferably the cell is a cell of the human immune system.

### Antibodies

Antibodies may be produced by standard techniques, such as by immunisation using the polypeptides, polynucleotides, fixed cells and pharmaceutical compositions of the present invention or by using a phage display library.

For the purpose of this invention, the term "antibody", unless specified to the contrary, includes but is not limited to, polyclonal, monoclonal, chimeric, single chain, Fab fragments, fragments produced by a Fab expression library, as well as mimetics thereof. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. Furthermore, the antibodies and fragments thereof may be humanised antibodies. Neutralising antibodies, i.e., those which inhibit biological activity of the substance polypeptides, are especially preferred for diagnostics and therapeutics.

If polyclonal antibodies are desired, a selected mammal (e.g., mouse, rabbit, goat, horse, etc.) is immunised with the agents e.g., HIV envelope and CD4 receptor polynucleotides/polypeptides, fixed cells and pharmaceutical compositions of the present invention. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (*Bacilli Calmette-Guerin*) and *Corynebacterium parvum* are potentially useful human adjuvants which may be employed if purified the substance polypeptide is administered to immunologically compromised individuals for the purpose of stimulating systemic defence.

Serum from the immunised animal is collected and treated according to known procedures. If serum containing polyclonal antibodies to epitopes of the HIV-envelope-CD4 complex contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against the agents of the present invention can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; i.e., for isotype and epitope affinity.

Monoclonal antibodies to the agents of the present invention may be prepared using any technique which provides for the production of antibody molecules by continuous cell lines in culture. These include, but are not limited to, the hybridoma technique originally described by Koehler and Milstein (Koehler and Milstein, 1975), the human B-cell hybridoma technique (Kosbor et al., 1983; Cote et al., 1983) and the EBV-hybridoma technique (Cole et al., 1985). In addition, techniques developed for the production of "chimeric antibodies", the splicing of mouse antibody genes to human antibody genes to obtain a molecule with appropriate antigen specificity and biological activity can be used (Morrison et al., 1984; Neuberger et al., 1984; Takeda et al., 1985). Alternatively, techniques described for the production of single chain antibodies (US Patent No. 4,946,779) can be adapted to produce the substance specific single chain antibodies.

Within certain embodiments, the use of antigen-binding fragments of antibodies may be preferred. Such fragments include Fab fragments, which may be prepared using standard techniques, for example digestion by papain to yield Fab and Fc fragments which may be separated by affinity chromatography.

The ability of antibodies of the present invention to interfere with the physiological functions of CD4 on uninfected lymphocytes should be limited, since their binding affinity to the isolated CD4 molecule should be relatively low.

Modifications of the antibodies isolated according to the present invention may be made to improve their characteristics. For example, modifications may be made using affinity maturation through a mutation-prone, display library of the gene encoding for the variable part of the antibody, in order to select for mutants with higher specificity for CD4 epitope(s) strictly depending on gp120 binding (thus reducing the potential reactivity of the antibody with uninfected CD4-expressing cells). Phage display technology as described by McCafferty (McCafferty et al., 1990) can be used to produce human antibodies and antibody fragments in vitro from immunoglobulin variable (V) domain gene repertoires from unimmunized donors. According to this technique, antibody V domain genes are closed in-frame either into either a major or minor coat protein gene of a filamentous bacteriophage, such as M1 3 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Phage display can be performed in a variety of formats as reviewed by, for example, Johnson, et al., Curr. Opin. Struct. Biol. 3:564-571 (1993).

### Anti-idiotypic antibodies

In one aspect of the present invention there is provided anti-idiotypic antibodies to the antibodies of the present invention. Anti-idiotypic antibodies (anti-idotypes or "anti-ids") are antibodies directed against epitopes (called idiotypes) located in the antigen binding region or variable region of another antibody molecule. The "internal image" anti-idiotypic antibodies mimic the three-dimensional structure of the antigen (e.g., fixed cell) of the present invention. The administration of the anti-idiotypic antibody can induce the production of antibodies against the original antigen. Idiotype vaccines have been applied successfully on several transmissible diseases and in the treatment of cancer (for example: Uytdehaag and Osterhaus, 1986; Kennedy et al., 1986; Hiernaux, 1988; Stein and Soderstrom,1984).

Anti-idiotypic antibodies, in general, and their structural comparison with their antigens, and internal images, were investigated and discussed by several groups (e.g., Tsjisaki et al., 1993; Raychaudhuri, 1990; Bruck, 1986).

The present invention provides for anti-idiotypic antibodies which induce an immune response against the HIV envelop-CD4 complex. These antibodies can be used for diagnostic and immunological applications.

### Treatment

It is to be appreciated that all references herein to treatment include curative, palliative and prophylactic treatment of HIV and/or curative, palliative and prophylactic treatment of disorders related to the immune system. The treatment of mammals is particularly preferred. Both human and veterinary treatments are within the scope of the present invention.

### Immunotherapy

Similar conformational changes to those that occur when CD4 binds gp120 may be induced when CD4 binds other ligands, such as MHC class II, which is known to interact directly with CD4 during the process of antigen presentation to T cells, resulting in T-cell activation.

It follows that the antigens, pharmaceutical compositions and antibodies of the present invention may be used in the treatment of a disease or condition of, or related to the immune system. Preferably the antibody is DB-81 or a derivative thereof. In a preferred embodiment the disease is a T-cell mediated disease and more preferably a CD4+ T cell mediated disease. In another preferred embodiment the disease is an APC mediated disease.

CD4+ T cells recognise antigen which has been processed and is presented in association with a self class II MHC molecule. In initiating the immune response, this interaction takes place in the lymphoid tissue, and involves T cell interaction professional antigen presenting cells (which include B cells, macrophages and dendritic cells) which take up, process and present the relevant antigen. Once a CD4+ T cell has been activated in this way, it is capable of recognising the antigen presented by any cell which expresses the appropriate class II MHC molecule. In this way, initial activation of the immune response is controlled within the lymphoid tissues, whilst primed CD4+ T cells are then able to respond to antigen presented at distant sites within the body. Interactions of CD4 + T cells with antigen presenting cells (APC) lies at the root of the immune response.

Diseased or infectious states that may be described as being mediated by T cells include, but are not limited to, any one or more of asthma, allergy, tumour induced aberrations to the T cell system and infectious diseases such as those caused by Plasmodium species, Microfilariae, Helminths, Mycobacteria, HIV, Cytomegalovirus, Pseudomonas, Toxoplasma, Echinococcus, Haemophilus influenza type B, measles, Hepatitis C or Toxicara. Thus particular conditions that may be treated or prevented which are mediated by T cells include multiple sclerosis, rheumatoid arthritis and diabetes. The present invention may also be used in organ transplantation or bone marrow transplantation. The present invention is also useful in treating immune disorders such as autoimmune disorders or graft rejection such as allograft rejection.

Previous work has shown that treatment with antibodies specific for CD4 is effective in preventing a wide range of both experimentally-induced and spontaneously-occurring autoimmune diseases. For example, treatment with either anti-CD4 or anti-MHC class II antibodies was found to effectively prevent murine collagen-induced arthritis as well as murine streptococcal cell wall-induced arthritis (Ranges et al., 1985; Hom et al., 1988; Wooley et al., 1985; Cooper et al., 1988; Van den Broek et al., 1992). Anti-CD4 treatment also prevented systemic lupus erythematosus in NZB/NZW F1 (B/W) mice and BXSB mice (Wofsy et al., 1985; Wofsy et al., 1986; Ermak et al., 1989.

Examples of autoimmune disorders range from organ specific diseases (such as thyroiditis, insulitis, multiple sclerosis, iridocyclitis, uveitis, orchitis, hepatitis, Addison's disease, myasthenia gravis) to systemic illnesses such as rheumatoid arthritis or lupus erythematosus. Other disorders include immune hyperreactivity, such as allergic reactions.

In more detail, organ-specific autoimmune diseases include multiple sclerosis, insulin dependent diabetes mellitus, several forms of anemia (aplastic, hemolytic), autoimmune hepatitis, thyroiditis, insulitis, iridocyclitis, skleritis, uveitis, orchitis, myasthenia gravis, idiopathic thrombocytopenic purpura, inflammatory bowel diseases (Crohn's disease, ulcerative colitis).

Systemic autoimmune diseases include: rheumatoid arthritis, juvenile arthritis, scleroderma and systemic sclerosis, sjogren's syndrom, undifferentiated connective tissue syndrome, antiphospholipid syndrome, different forms of vasculitis (polyarteritis nodosa, allergic granulomatosis and angiitis, Wegner's granulomatosis, Kawasaki disease, hypersensitivity vasculitis, Henoch-Schoenlein purpura, Behcet's Syndrome, Takayasu arteritis, Giant cell arteritis, Thrombangiitis obliterans), lupus erythematosus, polymyalgia rheumatica, essentiell (mixed) cryoglobulinemia, Psoriasis vulgaris and psoriatic arthritis, diffus fasciitis with or without eosinophilia, polymyositis and other idiopathic inflammatory myopathies, relapsing panniculitis, relapsing polychondritis, lymphomatoid granulomatosis, erythema nodosum, ankylosing spondylitis, Reiter's syndrome, different forms of inflammatory dermatitis.

A more extensive list of disorders includes: unwanted immune reactions and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery or organ, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

### Pharmaceutical compositions

A pharmaceutical composition is a composition that comprises or consists of a therapeutically effective amount of a pharmaceutically active agent. It preferably includes a pharmaceutically acceptable carrier, diluent or excipients (including combinations thereof). Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Examples of pharmaceutically acceptable carriers include, for example, water, salt solutions, alcohol, silicone, wavers, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethylcellulose, polyvinylpyrrolidone, and the like.

Where appropriate, the pharmaceutical compositions can be administered by any one or more of: inhalation, in the form of a suppository or pe ssary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

### Vaccines

The preparation of vaccines which contain an immunogenic polypeptide(s)/polynucleotide(s) as active ingredient(s), is known to one skilled in the art. Typically, such vaccines are prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof.

In addition, if desired, the vaccine may contain minor amounts of auxiliary substance such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion.

Further examples of adjuvants and other agents include aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, *Corynebacterium parvum* (*Propionobacterium acnes*), *Bordetella pertussis*, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

Typically, adjuvants such as Amphigen (oil-in-water), Alhydrogel (aluminum hydroxide), or a mixture of Amphigen and Alhydrogel are used. Only aluminum hydroxide is approved for human use.

The proportion of immunogen and adjuvant can be varied over a broad range so long as both are present in effective amounts. For example, aluminum hydroxide can be present in an amount of about 0.5% of the vaccine mixture (Al₂O₃ basis). Conveniently, the vaccines are formulated to contain a final concentration of immunogen in the range of from 0.2 to 200 µg/ml, preferably 5 to 50 µg/ml, most preferably 15 µg/ml.

The effectiveness of an adjuvant may be determined by measuring the amount of antibodies directed against an immunogenic agent resulting from administration of this agent in vaccines which are also comprised of the various adjuvants.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer

Further preferred features and embodiments of the present invention will now be described by way of non-limiting example and with reference to the accompanying drawings in which:
Figure 1 shows a graphical output of a cytofluorimetric analysis using uninfected NIH-3T3 cells (panel A) or vCB43-infected NIH-3T3 cells in the presence or absence of sCD4 (panel B) as a target for serum IgG.
Figure 2 shows a graphical output of an ELISA assay using recombinant HIV-1 gp120 from a prototypic R5 isolate (JR-FL) or from a prototypic X4 isolate (IIIB), either alone or in combination with 2D-sCD4 as a target for serum IgG.
Figure 3 shows a plot of cell fusion activity, using NIH-3T3 mouse cells infected with vCB43 as effector cells and HeLa human cells infected with a vaccinia expressing human CCR5 (vCCR5) plus CD4 (vCB3) as a target, versus serum dilution.
Figure 4 shows a plot of cell fusion activity, using target cells expressing Env from different HIV-1 subtypes, versus serum dilution
Figure 5 shows a plot comparing cell fusion activity of serum IgG and serum IgG which has been pre-adsorption with human sCD4.
Figure 6 shows ELISA analysis of binding of monoclonal antibody DB-8 1 to solid phase 2D-sCD4 used for coating alone or in equimolar complexes with recombinant gp120 from different HIV-1 isolates (JR-FL, CM235, IIIB, as indicated). The amount of antibody is indicated on the *x*-axis, optical density on the *y*-axis.
Figure 7 shows cell fusion inhibition by mAb DB-81. The amount of antibody is indicated on the *x*-axis, cell fusion expressed as percent of control (i.e., without antibody added) on the *y*-axis. In the cell fusion assay, vaccinia recombinants were used.

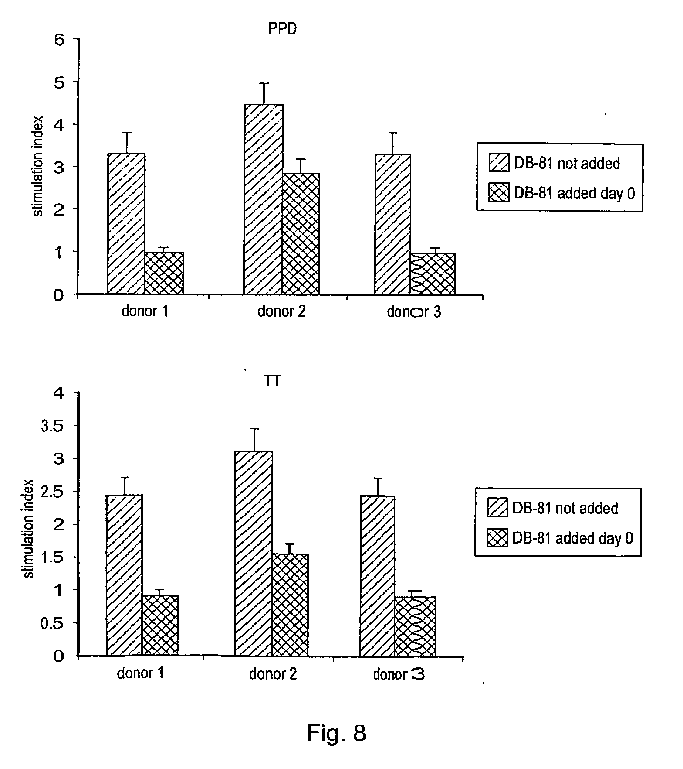

Figure 8 shows the effect of DB-81 mAB addition (5 µg/ml) to Purified Protein Derivates from Mycobacterium tuberculosis (PPD, top panel) or tetanus toxoid (TT, bottom panel) stimulated peripheral blood mononuclear cells (PBMC) from 3 healthy donors, in a standard 5-days proliferation assays. Proliferation was measured in a 96 well flat bottom plate microculture system, as 3H Thymidine (3H-TdR) incorporation, following pulse of cells with 0,5 µCi 3H-TdR per well in the last 18 hours of incubation. Bars indicate standard deviation of quadruplicates. Results are expressed as stimulation index, which represents the ratio between the proliferation observed in test wells versus that observed in unstimulated control wells.
Figure 9 shows a plot comparing the effect of sera from mice inoculated with a vaccinia vector encoding gp160 BaL (vCB43), a vector encoding soluble CD4 (vCB5) or both vectors simultaneously (vCB43 + vCB5) on cell fusion induced by different HIV-1 envelopes.

Table 1 shows the characterisation of IgG binding activity to the indicated solid phase antigens in supernatants from fusion of spleen cells from mouse immunised with vCB43 infected, 2D-sD4 reacted NIH-3T3 cells; Binding activity of 1/2 diluted supernants is shown, after 30 min developing time. Values are qualitative, since different numbers of cells were present in different microcultures. Asterisks indicate supernatants where an increased binding to the CD4/gp120 complex was observed.

The vaccinia vectors used in the examples were obtained from Dr. Edward A. Berger of the Laboratory of Viral Diseases, NIAID, NIH, Bethesda, MD, under MTA. Soluble sCD4 protein was obtained from the NIH AIDS Reagent Program (Rockville, MD).

### Example 1 - Preparation of fixed cells

NIH-3T3 cells were infected with a vaccinia vector expressing the HIV-1 BaL envelope (vCB43), then treated with 2D-sCD4 and fixed with glutaraldehyde. The fixation was done by incubating cells (previously reacted with 1µg soluble CD4 per million cell), at a 2 x 10⁶ cells per ml concentration with glutaraldehyde 0,5 %, for 10 min at 20 °C. Glutaraldehyde was prepared from grade I aqueous solution 25% (Sigma G5882) and diluted with phosphate buffered saline (PBS) up to final concentration. Cells were then washed 3 times with 10 ml ice cold PBS (1,500 RPM per 5 min each time) and brought in PBS to a concentration suitable for injecting the immunogen (500,000 cells per mouse per injection in 250 µl injection volume).

### Example 2 - Generation of serum IgG which preferentially recognises gp120-sCD4 complex

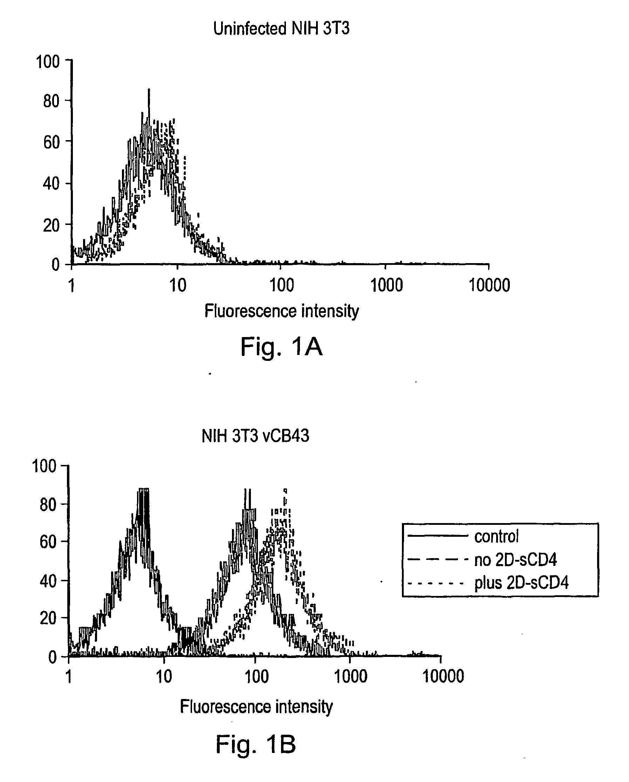

Serum IgG obtained from mice injected subcutaneously with cells expressing a complex of gp120 and sCD4 on their surface preferentially recognize gp120 when complexed with sCD4, as shown by cytofluorimetric analysis (Figure 1). Pooled sera from 4 mice immunized with NIH-3T3 cells infected with a vaccinia vector expressing the HIV-1 BaL envelope (vCB43), then treated with 2D-sCD4 and fixed with glutaraldehyde were used.

As a target for cytofluorimetric analysis uninfected (panel A) or vCB43-infected (panel B) NIH-3T3 cells were used. Solid lines represent the background fluorescence profile (cells reacted with FITC-conjugated, goat-anti-mouse IgG polyclonal antibody). Binding to cells treated (dotted line) or not (dashed line) with 2D-sCD4 are shown. The cells were analysed on a FACScan analyser.

### Example 3 - Generation of serum IgG which preferentially recognises solid phase recombinant HIV-1 gp120 solid phase antigen

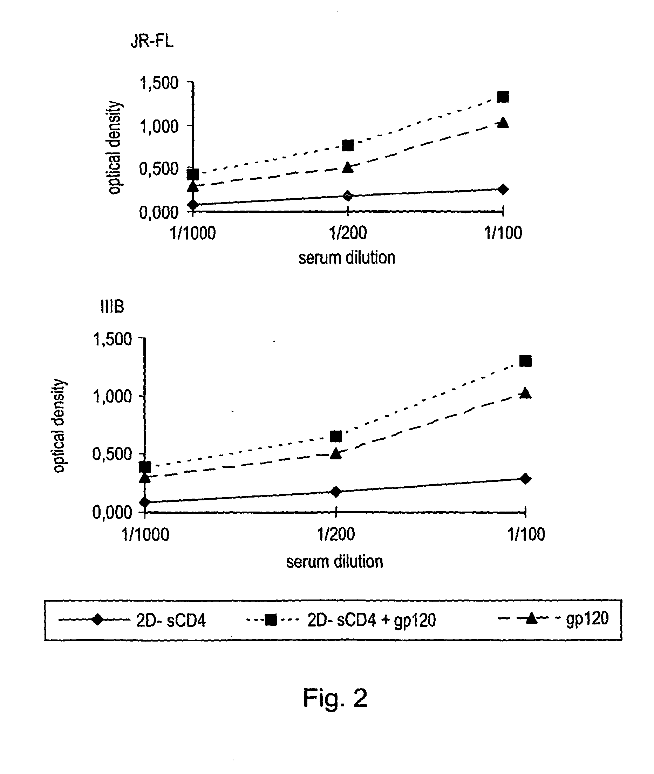

Serum IgG obtained from mice injected subcutaneously with cells expressing a complex of gp120 and sCD4 on their surface preferentially react with gp120/sCD4 complexes, as shown by ELISA assay (Figure 2). Pooled sera from 4 injected subcutaneously with NIH-3T3 cells infected with a vaccinia vector expressing the HIV-1 BaL envelope (vCB43), treated with 2D-sCD4 and fixed with glutaraldehyde were used. The solid phase antigen was recombinant HIV-1 gp120 from a prototypic R5 isolate (JR-FL) or from a prototypic X4 isolate (IIIB), used either alone or in combination with 2D-sCD4. The ELISA assay was performed using standard protocols.

### Example 4 -Inhibition of HIV-1 envelope-mediated cell fusion

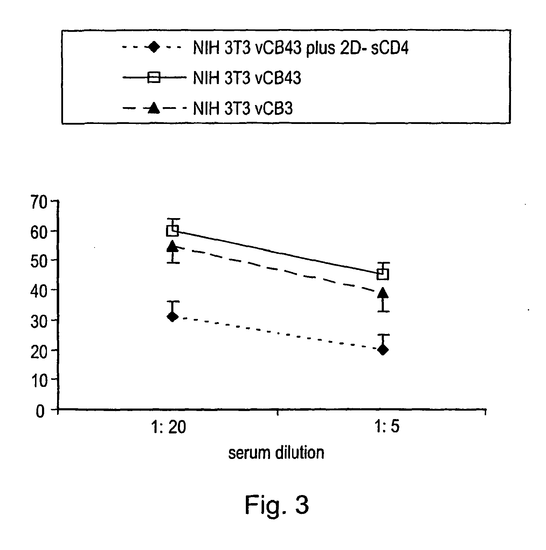

HIV-1 envelope-mediated cell fusion is inhibited by pooled sera obtained from mice injected with NIH-3T3 cells expressing the HIV-1 BaL envelope (vCB43) and then treated with 2D-sCD4 and fixed with glutaraldehyde (Figure 3). Sera from mice injected with cells expressing the HIV-1 envelope but not complexed with sCD4 were used as a control. To assess the relevance of anti-human CD4 antibodies to the observed neutralisation, sera from mice injected with a vaccinia vector expressing human CD4 (vCB3) were tested in parallel as a control. The cell fusion assay was performed as described (Feng et al. *Science* 1996) using NIH-3T3 mouse cells infected with vCB43 as effector cells and HeLa human cells infected with a vaccinia expressing human CCR5 (vCCR5) plus CD4 (vCB3) as a target. Effector cells were co-infected with a vaccinia vector expressing bacteriophage T7 RNA polymerase; target cells with a vaccinia vector expressing the *lacZ* reporter gene under the control of the T7 promoter. Cell fusion was determined by measurement of β-galactosidase activity in non-ionic detergent cell lysates. The sera, previously heat-inactivated, were added to target cells prior to the fusion assay at the dilution indicated. The results are expressed as percent fusion activity relative to the positive control (no inhibitor added).

### Example 5 - Inhibition of cell fusion mediated by diverse HIV-1 envelopes

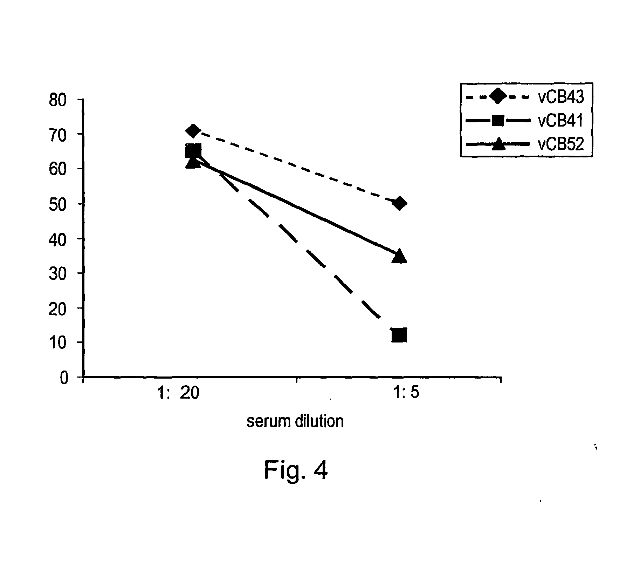

Pooled sera from mice injected with NIH-3T3 cells expressing the HIV-1 BaL envelope (vCB43), treated with 2D-sCD4 and fixed with glutaraldehyde inhibit cell fusion mediated by diverse HIV-1 envelopes (Figure 4). Vaccinia recombinants encoding Env from different HIV-1 subtypes and with different coreceptor usage were used: vCB52, strain CM235 (Subtype A/E (Circulating Recombinant Form CRF01-AE); CCR5-dependent); vCB43, strain BaL (subtype B; CCR5-dependent); vCB41, strain LAV (subtype B; CXCR4-dependent).

### Example 6 - Immune sera with CD4 independent activity

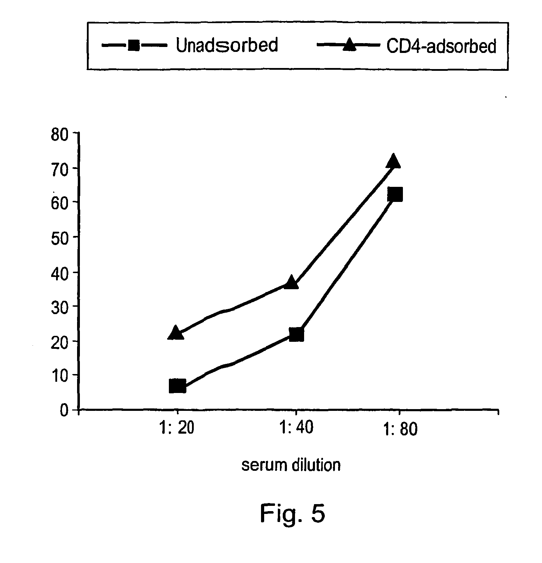

Pre-adsorption with human CD4 does not abrogate the inhibitory activity of immune sera on HIV-1 envelope-mediated fusion (Figure 5). Pooled sera from 4 mice injected with NIH-3T3 cells expressing the HIV-1 BaL envelope (vCB43) and treated with 2D-sCD4 and fixed with glutaraldehyde were pre-adsorbed on Sub-B1 cells expressing high levels of human CD4. A drastic reduction of CD4 reactivity was observed by ELISA.

### Example 7 - Antibody isolation

The screening of antibodies was performed with the specific aim of identifying antibodies capable of binding the CD4/gp120 complex with enhanced affinity, as compared with the binding affinity to each moiety of the complex (i.e., CD4 or gp120) taken individually. The rationale behind this work is that antibodies with these characteristics should effectively interfere with HIV infection at the post-binding level, i. e. after the initial interaction of HIV with the CD4 receptor on the surface of the target cells, preventing subsequent steps in the viral entry process.

Fusion of spleen cells from mouse immunised with vCB43 infected, 2D-sCD4 reacted NIH-3T3 cells (fixed cells) was performed with polyethylene glycol solution, 50% vol/vol in RPMI without serum, mol wt of PEG 1,450. Fused cells (Sp2/0-Ag14 mouse, Balb/b, hybrid, myeloma fused with splenocytes from immunized Balb/c mice) were plated at 2 x 10⁶ cell dose per well in 24 well plate, in selection medium (i.e., complete RPMI-10% FCS medium containing also HAT, i.e. hypoxanthine 100 microM, aminopterin 800 nM, thymidine 16 microM). Fifteen days later cells supernatant (1 to 2 x diluted in PBS containing 0,05 % Tween 20 as detergent) of each culture were separately tested for IgG binding activity by means of ELISA with three different solid-phase antigens coated onto 96 wells microplates: soluble 4 domain CD4 (50,000 mol wt, 1 microg per ml in PBS coating, done at 4° C, ON), soluble gp120 (120,000 mol wt, 2,4 µg/ml, from BAL HIV-1 strain, same coating time and temperature) and the complex obtained by incubating, before coating, 30 min on ice, equimolar amounts of the two molecules (the, same coating time and temperature were applied). ELISA wells were blocked (1 h at RT) with PBS containing NaN3 0,02% and bovine serum albumine 1%. As a probe, an affinity purified alkaline phosphatase conjugated goat anti mouse polyclonal antibody specific to the Fc fragment of mouse IgG was used, at a 1 microgram per ml concentration (1 hr, RT). Dilution of the probe (starting concentration: 1 mg/ml) was done with PBS containing 1% bovine serum albumine. pNPP (4-Nitrophenyl phosphate disodium salt) was used as a substrate for developing the reaction, which was stopped with 3N NaOH and read at 405 nM when control wells (medium only) were still visually uncoloured (O.D. < 0,1). This usually corresponded to 30 min developing time. Washings between each step of the procedure were done with PBS containing Tween-20 0,05% and NaN3 0,02%, by using an automated ELISA washer (5 steps washing-program). Supernatants whose IgG binding activity yielded an optical density above 0,5 O.D. after 30 min developing at 405 nM reading, when the negative control (medium only) was below 0,1 O.D., in ELISA wells coated with the complex were identified. Among them, only supernatants whose IgG binding activity to the complex was higher as compared to binding to CD4 or to gp120, were selectively expanded in HT medium (containing hypoxanthine 100 µM, thymidine 16 µM) for 20 days: IgG binding activity to complex was re-tested in these supernatants and, where appropriate (i.e. when binding activity to the complex was confirmed to be above binding to each single molecule), were cloned at 0,2 cells per well in complete medium containing HT and Hybridoma Enhancing Supplement Hybri-Max (Sigma). IgG binding activity of the clones isolated is shown in Table 1. Single clones were gradually adapted to grow in complete medium without HT and without Hybridoma Enhancing Supplement. All ELISA reagents were obtained from NIH AIDS Reagent Program. All cell culture reagents were from Sigma.

Antibody DB-81 has been extensively evaluated *in vitro* for its capability to interfere with HIV-1-dependent cell fusion using cell lines expressing envelopes from biologically diverse viral isolates, as well as with HIV-1 replication in primary peripheral blood cells. In both cases, DB-81 has revealed a strong capability to interfere with HIV-1 function. The hybridoma cell line producing DB-81 was deposited at the Advanced Biotechnology Center Inter Lab Cell Line Collection (CBA ICLB) on 5 August 2003.

### Example 8- Specificity of DB-81 to CD4/Env complex

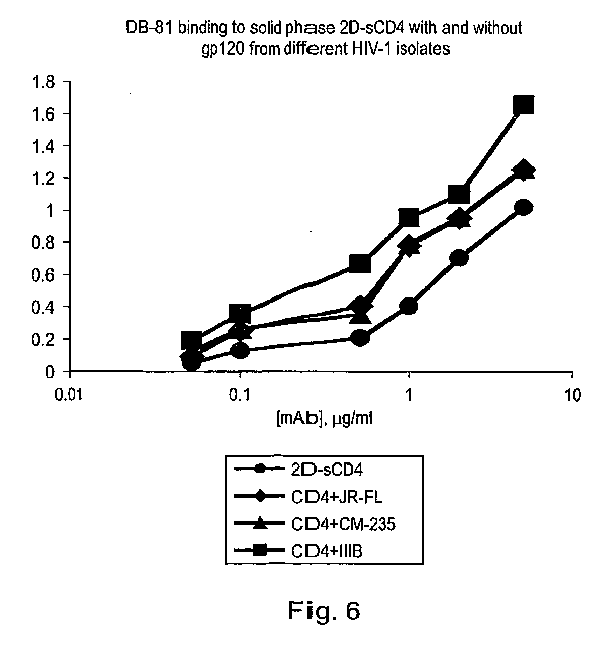

The specificity of antibody DB-81 to CD4 and CD4/Env complexes was determined by ELISA analysis. ELISA assays were performed using solid phase recombinant 4-domain (4D) or 2-domain (2D) soluble CD4 (2D- and 4D-sCD4). Binding to both 2D-and 4D-sCD4 was equivalent. The binding of DB-81 to solid phase 2D-sCD4 alone or in equimolar complexes with recombinant gp120 (following pre-incubated for 30 minutes in ice) from different HIV-1 isolates (JR-FL, CM235, HIB). Representative results are shown in Figure 6.

### Example 9 - Ability of DB-81 to interfere with HIV-1-dependent cell fusion

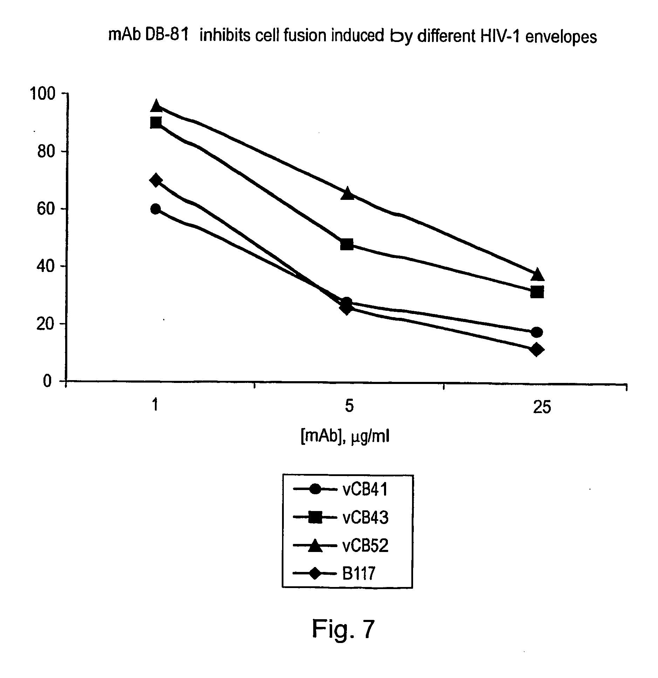

Effector cells (HeLa) infected with vaccinia vectors expressing biologically different HIV-1 envelopes [a prototypic wild-type R5 (macrophage-tropyc, non syncyium-inducing) Env (vCB-43, Ba-L), a primary R5 clade-E cloned envelope (vCB52), a laboratory-adapted R4 Env (vCB-41, LAV) or a primary multi-tropic R5-X4-R3 envelope (B 117)] were mixed for 2 hrs with target cells infected with vaccinia vectors expressing the appropriate coreceptor (NIH3T3-CCR5 or HeLa-CXCR4) and CD4. Effector cells were always co-infected with a vaccinia vector expressing bacteriophage T7 RNA polymerase; target cells with a vaccinia vector expressing the *lacZ* reporter gene under the control of the T7 promoter. Cell fusion was determined by measurement of β-galactosidase activity in non-ionic detergent cell lysates. As expected, cell fusion occurred only when cells expressing the proper Env/coreceptor pairs were mixed, in the presence of 2D-sCD4. Using this cell fusion protocol, the effects of DB-81 at different concentrations was evaluated. Representative results are shown in Figure 7. It can be seen from Figure 7 that the mAb was consistently more effective against the 2 CXCR4-using strains (regardless of previous adaptation to laboratory growth) than against the 2 CCR5-dependent isolates.

### Example 10 - Anti-inflammatory properties of mAb DB-81

Since mAb DB-81 may be specific for a CD4 epitope induced upon binding of a specific ligand (in our instance, gp120 of HIV), it is plausible that similar conformational changes might be induced by other ligands, such as MHC class II, which is known to interact directly with CD4 during the process of antigen presentation to T cells, resulting in T-cell activation. Interference with this interaction or with the CD4-class II complex may result in defective T-cell activation and thereby anti-inflammatory effects. To verify the potential anti-inflammatory effects of mAb DB-81, we investigated its able to interfere with the process of antigen-specific T-cell activation. The results are shown in Figure 8. Antigen specific proliferation to Purified Protein Derivatives (PPD) and tetanus toxoid (TT) was inhibited in peripheral blood mononuclear cells from 3 different individual, when DB-81 mAb was added at time 0, in a standard 5-days 3HTdR -T cell proliferation assay. Proliferation was measured in a 96 well flat bottom plate microculture system, as 3H Thymidine (3H-TdR) incorporation, following pulse of cells with 0,5 µCi 3H-TdR per well in the last 18 hours of incubation. Bars indicate standard deviation of quadruplicates. Results are expressed as stimulation index, which represents the ratio between the proliferation observed in test wells versus that observed in unstimulated control wells.

### Example 11 - In-vivo vaccination

Animal cells may be transduced *in vivo* with a polynucleotide encoding an HIV-1 envelope protein and a polynucleotide encoding an sCD4 receptor, resulting in expression of native oligomeric HIV-1 envelope protein on the surface of transduced cells, as well as the local release of sCD4 receptor. The sCD4 receptor binds to the expressed envelope protein generating an immunogenic complex in-vivo.

The formation of multimolecular complexes between native HIV envelope and sCD4 may be obtained in vivo by co-inoculation of two separate viral vectors, one expressing gp160 and another expressing soluble CD4. Efficient expression of both proteins can be monitored using specific monoclonal antibodies; the effective formation of gp120/CD4 complexes on the cellular membrane can be verified using complex-specific antibodies, including but not limited to 17b, 48d, D1 9. Independent expression of the two proteins in the same or, more likely, in different target cells leads to the release of sCD4 that will in turn bind to the cell-surface expressed gp120/gp41 oligomeric complexes, triggering the CD4-induced envelope conformation.

Inoculation was performed by intradermal injection in the footpad, 100µl of phosphate buffer containing 5x10⁶ pfu per mouse of the vaccinia vector vCB43 (expressing gp160 from the R5 strain, BaL) or vCB5 (expressing soluble CD4), alone or in combination, both grown in HeLa cells. After coinoculation of the two vaccinia vectors, the simultaneous expression of both gp120 and sCD4 was observed in mouse tissues, as was the spontaneous formation of gp120/sCD4 complexes, as revealed by immunostaining with complex-specific anti-gp120 antibodies that fail to recognise unbound gp120.

Sera from mice coinoculated with the 2 vectors were tested for their ability to recognise either gp120, sCD4 or the gp120/sCD4 complex in ELISA, showing an increased binding to the complex, compared to each molecule alone.

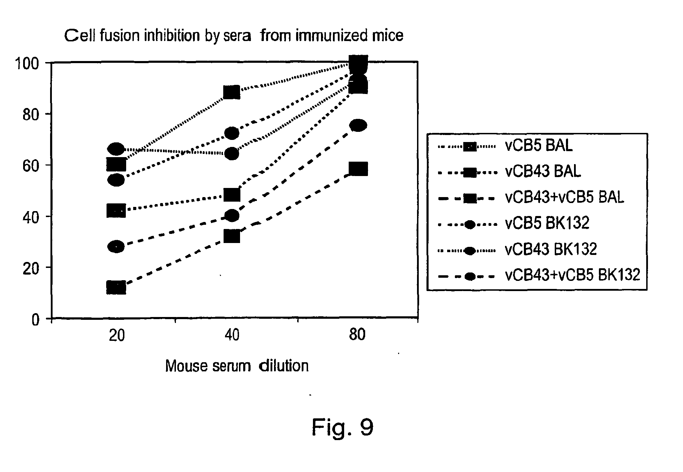

The same sera were tested for their ability to neutralise both the homologous and heterologous HIV-1 isolates. In both cases, the sera from mice immunised with the 2 vectors showed an increased ability to block infection by both homologous and heterologous isolates, compared to sera from animals immunised with each vector individually. Of note, extensive pre-adsorption of sera on CD4-positive cells, in order to remove all the reactivity against CD4, failed to significantly reduce the neutralising activity of the sera. Representative results are shown in Figure 9.

Various modifications and variations of the describ ed methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments.

**Table 1.**

| well | binding to CD4 | binding to BAL gp120 | binding to CD4/ BAL |
|---|---|---|---|
| DB1 | 2.203 | 0.045 | 1.321 |
| DB 2 | 2.342 | 0.035 | 1.221 |
| DB 3 | 3.222 | 0.032 | 0.982 |
| DB 9 | 0.040 | 2.121 | 2.001 |
| DB 10 | 0.045 | 2.980 | 2.322 |
| DB 11 | 0.034 | 1.342 | 1.032 |
| DB 12 | 0.098 | 1.323 | 1.120 |
| DB 4 | 0.043 | 0.430 | 0.462 |
| DB 6 | 0.087 | 0.324 | 0.231 |
| DB 6 | 0.087 | 0.342 | 0.231 |
| DB 12 | 2.120 | 0.131 | 2.241 |
| DB 1 | 1.320 | 0.011 | 1.232 |
| DB 2 | 1.983 | 0.198 | 1.763 |
| DB 3 | 1.348 | 0.022 | 1.134 |
| DB 8* | 1.221 | 0.045 | 1.430 |
| DB 81 * | 1.234 | 0.023 | 1.998 |
| DB 10* | 1.432 | 0.042 | 1.743 |
| DB 11* | 1.565 | 0.079 | 2.211 |
| DB 12* | 0.092 | 1.211 | 1.243 |
| DB 1 | 0.102 | 0.942 | 0.872 |
| DB 2 | 0.023 | 1.234 | 1.356 |
| DB 3 | 0.113 | 0.534 | 0.435 |
| DB 10* | 1.234 | 0.045 | 2.311 |
| DB 11 * | 1.342 | 0.023 | 2.432 |
| DB 12* | 1.532 | 0.065 | 2.543 |

The characterisation of IgG binding activity to the indicated solid phase antigens in supernatants from fusion of spleen cells from mouse immunised with vCB43 infected, 2D-sD4 reacted NIH-3T3 cells; Binding activity of 1/2 diluted supernants is shown, after 30 min developing time. Values are qualitative, since different numbers of cells were present in different microcultures. Asterisks indicate supernatants where an increased binding to the CD4/gp120 complex was observed.

### References

Allan et al. (1985) Science 228:1092
Kowalski et al. (1987) Science 237:71
Yang et al. (2002) J. Virol 76:4634
Veronese et al (1985) Science 229:1404
Thali et al. (1993) J. Virol. 67:3978
Sattentau et al. (1991) J. Exp. Med. 174:407
Mbah et al., J. Virol. 2001 75:7785
Lusso and Burastero, unpublished data
Celada et al. (1990) J. Exp. Med. 172:1143
Kang et al. (1994) J. Virol. 68:5854
DeVico et al. (1995) Virology 211:583
Fouts et al (2000) J. Virol. 74:11427
Yang et al. (2001) J. Virol. 75:1165
Berger (1988) Proc Natl Acad Sci U S A 85:2357
Sattentau et al. (1995) J. Exp. Med 182:185
Nyambi et al (1998) J. Virol. 72:9384
LaCasse et al. (1999) Science 283:357
Scarlatti et al. (1997) Nature Med. 3:1259-65.
Kwong et al (1998) Nature 393:648;
Kwong et al. (2000) J. Virol. 74:1961
Kwong et al., (2000) Structure Fold. Des. 8:1329
Schulke et al. (2002) J. Virol 76:7760
Berger (1997) AIDS 11(suppl. A):S3-S16
Berger et al. (1998) Nature 391:240
Weissenhom et al. (1997) Nature 391:240
Chan et al. (1997) Cell 89:263
Chan et al. (1998) Cell 93:681
Bleul et al. (1996) Nature 382:829
Murphy (1996) Cytokine Growth Factor Rev. 7:47
Yang et al. (2002) J.Virol 76:4634
Earl et al. (2002) J.Virol, 76:7760
Maddon et al. (1985) Cell 42:93
Peterson and Seed (1988) Cell 54:65
Arthos et al. (1989) Cell 57:469
Camerini and Seed (1990) Cell 60:747
Ohki et al. (1990) AIDS 4:1160
Lifson et al. (1991) AIDS Res. Hum. Retroviruses 7:521
Truneh et al. (1991) J. Biol. Chem. 266:5942
Vita et al. (1999) Proc. Natl. Acad Sci. U S A 96:13091
Vita et al. (2003) Nat Biotechnol. Jan;21(1):71-6.
Meyer et al. (1991) J. Gen. Virol. 72:1031
Smith and Moss (1983) Gene 25:21
MacLean et al. (1991) J. Gen. Virol. 72:632
Chou and Roizman (1992) PNAS 89:3266
Chou et al. (1994) J. Virol. 68:8304
Rice and Knipe (1990) J. Virol. 64:1704
Sullivan et al. (1998) J. Virol. 72:4694
Koehler and Milstein (1975) Nature 256:495-497
Kosbor et al. (1983) Immunol Today 4:72
Cote et al. (1983) Proc. Natl. Acad. Sci. 80:2026-2030
Cole et al. (1985) Monoclonal Antibodies and Cancer Therapy, Alan R Liss Inc, pp 77-96
Morrison et al. (1984) Proc. Natl. Acad. Sci. 81:6851-6855
Neuberger et al. (1984) Nature 312:604-608
Takeda et al. (1985) Nature 314:452-454
McCafferty et al. (1990) Nature 348:552-553
Johnson et al. (1993) Curr. Opin. Struct. Biol. 3:564-571
Uytdehaag and Osterhaus (1986) J.Immunol.134:1225
Kennedy et al. (1986) Science 232:220
Stein and Soderstrom (1984) J.Exp.Med. 160:1001
Tsjisaki et al. (1993) J. Immunol. 150:508
Raychaudhuri et al. (1990) J. Immunol. 145:760;
Bruck et al. (1986) Proc. Natl. Acad. Sci. USA 83:6578
Ranges et al. (1985) J. Exp. Med. 162: 1105-1110
Hom et al. (1988) Eur. J. Immunol. 18: 881-888
Wooley et al. (1985) J. Immunol. 134: 2366-2374
Cooper et al. (1988) J. Immunol. 141: 1958-1962
Van den Broek et al. (1992) Eur. J. Immunol. 22: 57-61
Wofsy et al. (1985) J. Immunol. 134: 852-857
Wofsy et al. (1986) J. Immunol. 136: 4554-4560
Ermak et al. (1989) Laboratory Investigation 61: 447-456

Applicant's or agent's file reference | P014017WOP CLM | International application No. PCT / IB 03 / 0 3 6 6 5

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 45 , line 2-4 .

B. IDENTIFICATION OF DEPOSIT | Further deposits are identified on an additional sheet [ ]

Name of depositary institution
Advanced Biotechnology Center (ABC)

Address of depositary institution (including postal code and country)
Interlab Cell Line Collection
(Biotechnology Dept)
Largo Rossana Benzi, 10
16132 Genova
Italy

Date of deposit | Accession Number
25 July 2003 | PD03002

C. ADDITIONAL INDICATIONS (leave blank if not applicable) | This information is continued on an additional sheet [ ]

In respect of those designations where a European Patent is sought, a sample of the deposited
microorganism will be made available until the publication of the mention of the grant of the European
Patent or until the date on which the application has been refused or withdrawn, only by issue of such a
sample to an expert nominated by the person requesting the sample.

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g. "Accession Number of Deposit")

For receiving Office use only | For International Bureau use only
[ ] This sheet was received with the international application | [ ] This sheet was received by the International Bureau on:
Authorized officer | Authorized officer

Form PCT/RO/134 (July1998)

## Claims

1. A pharmaceutical composition comprising (i) a vector comprising a polynucleotide encoding a gp160 HIV envelope protein and (ii) a vector comprising a polynucleotide encoding a CD4 receptor, wherein the vectors are independently selected from the group comprised of a lentivirus, a murine leukemia virus, a herpes virus, a mouse mammary tumor virus, a rous sarcoma virus, a fujinami sarcoma virus, an FBR murine osteosarcoma virus, a moloney murine sarcoma virus, an avian myelocytomatosis virus - 29 and an avian erythroblastosis virus.

2. A pharmaceutical composition according to claim 1 wherein (i) and (ii) are encoded on separate vectors.

3. A pharmaceutical composition according to claim 1 wherein (i) and (ii) are encoded on the same vector.

4. A pharmaceutical composition according to any one of the preceding claims wherein said HIV envelope protein and said CD4 receptor are expressed as separate proteins.

5. A pharmaceutical composition comprising (i) a polynucleotide encoding a gp160 HIV envelope protein and (ii) a CD4 receptor polypeptide.

6. A pharmaceutical composition according to any one of the preceding claims wherein the HIV envelope protein is expressed as part of a fusion protein and/or the CD4 receptor is expressed as part of a fusion protein.

7. A pharmaceutical composition according to any one of the preceding claims wherein the CD4 receptor is soluble CD4 receptor.

8. A pharmaceutical composition according to any one of the preceding claims wherein the CD4 receptor is human CD4 receptor.

9. A pharmaceutical composition according to any one of the preceding claims wherein the CD4 receptor is a CD4 surrogate molecule.

10. A pharmaceutical composition according to claim 9 wherein the CD4 surrogate molecule is M33 mini-protein.

11. A pharmaceutical composition according to any one of the preceding claims wherein the HIV envelope protein is HIV-1 envelope protein.

12. A pharmaceutical composition according to any one of the preceding claims wherein the HIV envelope protein is CCR5-dependent HIV envelope protein.

13. A pharmaceutical composition according to any one of the preceding claims further comprising a pharmaceutically acceptable diluent or excipient.

14. A pharmaceutical composition according to any one of the preceding claims for use in the treatment of HIV.

15. A pharmaceutical composition according to any one of claims 1-14 in the form of a vaccine.

16. Use of a pharmaceutical composition as defined in any one of claims 1-14 for the preparation of a medicament for treating or preventing HIV infection.

17. Use of a pharmaceutical composition comprising (i) a polynucleotide encoding a gp160 HIV envelope protein and (ii) a polynucleotide encoding a CD4 receptor for the preparation of a medicament for treating or preventing HIV infection wherein the administration of the pharmaceutical composition is in proximity of a superficial lymph-node station.

18. A fixed cell wherein said cell comprises an HIV envelope complex encoded by *gp160* and further wherein said envelope is complexed with a CD4 receptor.

19. A fixed cell according to claim 18 where the HIV envelope is as defined in any one of claims 6, 11 or 12 and wherein the CD4 receptor is as defined in any one of claims 7 to 10.

20. A fixed cell according to claim 18 or 19 wherein said envelope complex is an oligomeric gp120-gp41 complex.

21. A fixed cell according to any one of claims 18 to 20 wherein the fixed cell is derived from a T-cell.

22. A fixed cell according to any one of claims 18 to 21 wherein the fixed cell is fixed with glutaraldehyde.

23. A fixed cell according to any one of claims 18 to 22 for use in medicine.

24. Use of a fixed cell as defined in any one of claims 18 to 22 for the preparation of a medicament for treating or preventing HIV infection.

25. A vaccine comprising the fixed cell as defined in any one of claims 18 to 22.

26. Use of a fixed cell as defined in any one of claims 18 to 22 for generating anti-HIV monoclonal antibodies.

27. An antibody generated against the fixed cell as defined in any one of claims 18 to 22 wherein the antibody is immunospecific for the HIV envelope - CD4 complex.

28. An antibody according to claim 27 wherein said antibody is immunospecific for the CD4-gp120 complex.

29. An antibody according to claim 27 wherein said antibody has greater immunospecificity for said CD4-gp120 complex than either gp120 or CD4 alone.

30. An antibody according to any of claims 27 to 29 for use in medicine.

31. Use of an antibody as defined in any of claims 27 to 29 for the preparation of a medicament for treating or preventing HIV infection.

32. Use of a antibody as defined in any one of claims 27 to 29 for the preparation of a medicament for treating or preventing inflammation.

33. A hybridoma cell line having the identifying characteristics of the cell line deposited with the Advanced Biotechnology Center Inter Lab Cell Line Collection (CBA ICLB) wherein said hybridoma produces DB-81.

34. An antibody molecule produced by the hybridoma of claim 33.

35. An antibody that specifically binds the same idiotope as the antibody produced by the hybridoma of claim 33.

36. A method of generating a fixed cell according to the invention comprising
(i) expressing HIV envelope protein encoded by *gp160* on the surface of living cells;
(ii) complexing the envelope protein of (i) with a CD4 receptor; and
(iii) fixing the CD4 coated cells.

37. A method according to claim 36 wherein said HIV envelope protein is expressed on the surface of the living cell by transducing the cell with a vector encoding the HIV envelope protein.

38. A method according to claim 36 or 37 wherein CD4 receptor is expressed from the living cell.

39. A method according to claim 38 wherein the living cell expresses said HIV envelope protein and said CD4 receptor from a single vector.

40. A pharmaceutical composition comprising a fixed cell according to any one of claims 18 to 22 and a pharmaceutically acceptable diluent or excipient.

41. A pharmaceutical composition comprising a population of fixed cells according to any one of claims 18 to 22, wherein within the population more than one HIV envelope serotype is expressed, and a pharmaceutically acceptable diluent or excipient.

## Patentansprüche

1. Pharmazeutische Zusammensetzung mit (i) einem Vektor, der ein Polynukleotid umfasst, das ein gp160 HIV-Hüllprotein codiert, und (ii) einem Vektor, der ein Polynukleotid umfasst, das einen CD4-Rezeptor codiert, wobei die Vektoren unabhängig voneinander aus der Gruppe ausgewählt sind, bestehend aus einem Lentivirus, einem Maus-Leukämievirus, einem Herpesvirus, einem Mammatumorvirus der Maus, einem Rous-Sarkom-Virus, einem Fujinami-Sarkom-Virus, einem FBR-Osteosarkom-Virus der Maus, einem Moloney-Sarkom-Virus der Maus, einem Myelocytomatose-Virus-29 vom Vogel und einem Erythroblastose-Virus vom Vogel.

2. Pharmazeutische Zusammensetzung nach Anspruch 1, wobei (i) und (ii) auf getrennten Vektoren codiert werden.

3. Pharmazeutische Zusammensetzung nach Anspruch 1, wobei (i) und (ii) auf dem gleichen Vektor codiert werden.

4. Pharmazeutische Zusammensetzung nach einem der vorangegangenen Ansprüche, wobei das HIV-Hüllprotein und der CD4-Rezeptor als separate Proteine exprimiert werden.

5. Pharmazeutische Zusammensetzung mit (i) einem Polynukleotid, das ein gp160 HIV-Hüllprotein codiert und (ii) einem CD4-Rezeptor-Polypeptid.

6. Pharmazeutische Zusammensetzung nach einem der vorangegangenen Ansprüche, wobei das HIV-Hüllprotein als Teil eines Fusionsproteins exprimiert ist und/oder der CD4-Rezeptor als Teil eines Fusionsproteins exprimiert wird.

7. Pharmazeutische Zusammensetzung nach einem der vorangegangenen Ansprüche, wobei der CD4-Rezeptor löslicher CD4-Rezeptor ist.

8. Pharmazeutische Zusammensetzung nach einem der vorangegangenen Ansprüche, wobei der CD4-Rezeptor humaner CD4-Rezeptor ist.

9. Pharmazeutische Zusammensetzung nach einem der vorangegangenen Ansprüche, wobei der CD4-Rezeptor ein CD4-Surrogatmolekül ist.

10. Pharmazeutische Zusammensetzung nach Anspruch 9, wobei das CD4-Surrogatmolekül M33-Mini-Protein ist.

11. Pharmazeutische Zusammensetzung nach einem der vorangegangenen Ansprüche, wobei das HIV-Hüllprotein HIV-1-Hüllprotein ist.

12. Pharmazeutische Zusammensetzung nach einem der vorangegangenen Ansprüche, wobei das HIV-Hüllprotein CCR5-abhängiges HIV-Hüllprotein ist.

13. Pharmazeutische Zusammensetzung nach einem der vorangegangenen Ansprüche, weiche weiterhin ein pharmazeutisch verträgliches Verdünnungsmittel oder Füllmittel umfasst.

14. Pharmazeutische Zusammensetzung nach einem der vorangegangenen Ansprüche für die Verwendung bei der Behandlung von HIV.

15. Pharmazeutische Zusammensetzung nach einem der Ansprüche 1 bis 14 in der Form eines Impfstoffs.

16. Verwendung einer pharmazeutischen Zusammensetzung nach einem der Ansprüche 1 bis 14 für die Herstellung eines Medikaments zur Behandlung oder Verhinderung von HIV-Infektion.

17. Verwendung einer pharmazeutischen Zusammensetzung mit (i) einem Polynukleotid, das ein gp160-HIV-Hüllprotein codiert, und (ii) einem Polynukleotid, das einen CD4-Rezeptor codiert, für die Herstellung eines Medikaments zur Behandlung oder Verhinderung von HIV-Infektion, wobei die Verabreichung der pharmazeutischen .Zusammensetzung in der Nähe einer oberflächennahen Lymphknotenstelle durchgeführt wird.

18. Fixierte Zelle, wobei die Zelle einen HIV-Hüllkomplex umfasst, der von *gp160* codiert wird, und wobei die Hülle weiterhin mit einem CO4-Rezeptor komplexiert ist.

19. Fixierte Zelle nach Anspruch 18, wobei die HIV-Hülle wie in einem der Ansprüche 6, 11 oder 12 definiert ist und wobei der CD4-Rezeptor wie in einem der Ansprüche 7 bis 10 definiert ist.

20. Fixierte Zelle nach Anspruch 18 oder 19, wobei der Hüllkomplex ein oligomerer gp120-gp41-Komplex ist.

21. Fixierte Zelle nach einem der Ansprüche 18 bis 20, wobei die fixierte Zelle von einer T-Zelle abgeleitet ist.

22. Fixierte Zelle nach einem der Ansprüche 18 bis 21, wobei die fixierte Zelle mit Glutaraldehyd fixiert ist.

23. Fixierte Zelle nach einem der Ansprüche 18 bis 22 für die Verwendung in der Medizin.

24. Verwendung einer fixierten Zelle, wie sie in einem der Ansprüche 18 bis 22 definiert ist, für die Herstellung eines Medikamentes zur Behandlung oder Verhinderung von HIV-infektion.

25. Impfstoff, der die fixierte Zelle, wie sie in einem der Ansprüche 18 bis 22 definiert ist, enthält.

26. Verwendung einer fixierten Zelle, wie sie in einem der Ansprüche 18 bis 22 definiert ist, zur Erzeugung von monoklonalen Anti-HIV-Antikörpern.

27. Antikörper, der gegen die fixierte Zelle, wie sie in einem der Ansprüche 18 bis 22 definiert ist, erzeugt ist, wobei der Antikörper für den HIV-Hülle-CD4-Komplex immunspezifisch ist.

28. Antikörper nach Anspruch 27, wobei der Antikörper für den CD4-gp120-Komplex immunspezifisch ist.

29. Antikörper nach Anspruch 27, wobei der Antikörper eine höhere immunspezifität für den CD4-gp120-Komplex als für gp120 oder CD4 alleine hat.

30. Antikörper nach einem der Ansprüche 27 bis 29 für die Verwendung in der Medizin.

31. Verwendung eines Antikörpers, wie er in einem der Ansprüche 27 bis 29 definiert ist, für die Herstellung eines Medikaments zur Behandlung oder Verhinderung von HIV-infektion.

32. Verwendung eines Antikörpers, wie er In einem der Ansprüche 27 bis 29 definiert ist, für die Herstellung eines Medikaments zur Behandlung oder Verhinderung von Entzündung.

33. Hybridom-Zelllinie mit den Identifizierungsmerkmalen der Zelllinie, die bei der Advanced Biotechnology Center Interlap Cell Line Collection (CBA ICLB) hinterlegt ist, wobei das Hybridom DB-81 produziert.

34. Antikörpermolekül, hergestellt von dem Hybridom nach Anspruch 33.

35. Antikörper, der spezifisch das gleiche Idiotop wie der von dem Hybridom nach Anspruch 33 produzierte Antikörper bindet.

36. Verfahren zur Erzeugung einer fixierten Zelle gemäß der Erfindung, bei dem man
(i) HIV-Hüllprotein, codiert von *gp160,* auf der Oberfläche von lebenden Zellen exprimiert,
(ii) das Hüilprotein aus (i) mit einem CD4-Rezeptor komplexiert und
(iii) die mit CD4 überzogenen Zellen fixiert.

37. Verfahren nach Anspruch 36, wobei das HIV-Hüllprotein auf der Oberfläche der lebenden Zelle exprimiert wird, indem man die Zelle mit einem Vektor, der das HIV-Hüllprotein codiert, transduziert.

38. Verfahren nach Anspruch 36 oder 37, wobei der CD4-Rezeptor von der lebenden Zelle exprimiert wird.

39. Verfahren nach Anspruch 38, wobei die lebende Zelle das HIV-Hüllprotein und den CD4-Rezeptor von einem einzelnen Vektor exprimiert.

40. Pharmazeutische Zusammensetzung, die eine fixierte Zelle nach einem der Ansprüche 18 bis 22 und ein pharmazeutisch verträgliches Verdünnungsmittel oder Füllmittel umfasst.

41. Pharmazeutische Zusammensetzung mit einer Population von fixierten Zellen nach einem der Ansprüche 18 bis 22, wobei innerhalb der Population mehr als ein Serotyp der HIV-Hülle exprimiert wird, und mit einem pharmazeutisch verträglichen Verdünnungsmittel oder Füllmittel.

## Revendications

1. Composition pharmaceutique comprenant (i) un vecteur comprenant un polynucléotide codant pour une protéine d'enveloppe du VIH gp160 et (ii) un vecteur comprenant un polynucléotide codant pour un récepteur CD4, dans laquelle les vecteurs sont choisis indépendamment dans le groupe constitué d'un lentivirus, d'un virus de leucémie murine, d'un herpèsvirus, d'un virus de tumeur mammaire de souris, d'un virus de sarcome de Rous, d'un virus de sarcome de Fujinami, d'un virus d'ostéosarcome murin FBR, d'un virus de sarcome murin de Moloney, d'un virus de myélocytomatose aviaire 29 et d'un virus d'érythroblastose aviaire.

2. Composition pharmaceutique suivant la revendication 1, dans laquelle (i) et (ii) sont codés sur des vecteurs séparés.

3. Composition pharmaceutique suivant la revendication 1, dans laquelle (i) et (ii) sont codés sur le même vecteur.

4. Composition pharmaceutique suivant l'une quelconque des revendications précédentes, dans laquelle ladite protéine d'enveloppe du VIH et ledit récepteur CD4 sont exprimés sous forme de protéines séparées.

5. Composition pharmaceutique comprenant (i) un polynucléotide codant pour une protéine d'enveloppe du VIH gp160 et (ii) un polypeptide récepteur CD4.

6. Composition pharmaceutique suivant l'une quelconque des revendications précédentes, dans laquelle la protéine d'enveloppe du VIH est exprimée en tant qu'une partie d'une protéine de fusion et/ou le récepteur CD4 est exprimé en tant qu'une partie d'une protéine de fusion.

7. Composition pharmaceutique suivant l'une quelconque des revendications précédentes, dans laquelle le récepteur CD4 est un récepteur CD4 soluble.

8. Composition pharmaceutique suivant l'une quelconque des revendications précédentes, dans laquelle le récepteur CD4 est un récepteur CD4 humain.

9. Composition pharmaceutique suivant l'une quelconque des revendications précédentes, dans laquelle le récepteur CD4 est une molécule de substitution de CD4.

10. Composition pharmaceutique suivant la revendication 9, dans laquelle la molécule de substitution de CD4 est la mini-protéine M33.

11. Composition pharmaceutique suivant l'une quelconque des revendications précédentes, dans laquelle la protéine d'enveloppe du VIH est la protéine d'enveloppe du VIH-1.

12. Composition pharmaceutique suivant l'une quelconque des revendications précédentes, dans laquelle la protéine d'enveloppe du VIH est la protéine d'enveloppe du VIH dépendant de CCR5.

13. Composition pharmaceutique suivant l'une quelconque des revendications précédentes, comprenant en outre un diluant ou excipient pharmaceutiquement acceptable.

14. Composition pharmaceutique suivant l'une quelconque des revendications précédentes, destinée à être utilisée dans le traitement du VIH.

15. Composition pharmaceutique suivant l'une quelconque des revendications 1 à 14, sous forme d'un vaccin.

16. Utilisation d'une composition pharmaceutique telle que définie dans l'une quelconque des revendications 1 à 14, pour la préparation d'un médicament destiné au traitement ou à la prévention de l'infection par le VIH.

17. Utilisation d'une composition pharmaceutique comprenant (i) un polynucléotide codant pour une protéine d'enveloppe du VIH gp160 et (ii) un polynucléotide codant pour un récepteur CD4 pour la préparation d'un médicament destiné au traitement ou à la prévention de l'infection par le VIH, dans laquelle l'administration de la composition pharmaceutique est effectuée à proximité d'un emplacement de ganglion lymphatique superficiel.

18. Cellule fixée, ladite cellule comprenant un complexe d'enveloppe du VIH codé par *gp160* et en outre dans laquelle ladite enveloppe est complexée avec un récepteur CD4.

19. Cellule fixée suivant la revendication 18, dans laquelle l'enveloppe du VIH est telle que définie dans l'une quelconque des revendications 6, 11 et 12, et dans laquelle le récepteur CD4 est tel que défini dans l'une quelconque des revendications 7 à 10.

20. Cellule fixée suivant la revendication 18 ou 19, dans laquelle ledit complexe d'enveloppe est un complexe gp120-gp41 oligomère.

21. Cellule fixée suivant l'une quelconque des revendications 18 à 20, ladite cellule fixée étant dérivée d'un lymphocyte T.

22. Cellule fixée suivant l'une quelconque des revendications 18 à 21, ladite cellule fixée étant fixée avec du glutaraldéhyde.

23. Cellule fixée suivant l'une quelconque des revendications 18 à 22, destinée à être utilisée en médecine.

24. Utilisation d'une cellule fixée telle que définie dans l'une quelconque des revendications 18 à 22, pour la préparation d'un médicament destiné au traitement ou à la prévention de l'infection par le VIH.

25. Vaccin comprenant la cellule fixée telle que définie dans l'une quelconque des revendications 18 à 22.

26. Utilisation d'une cellule fixée telle que définie dans l'une quelconque des revendications 18 à 22, pour la production d'anticorps monoclonaux anti-VIH.

27. Anticorps engendré contre la cellule fixée telle que définie dans l'une quelconque des revendications 18 à 22, ledit anticorps étant immunospécifique pour le complexe enveloppe du VIH-CD4.

28. Anticorps suivant la revendication 27, ledit anticorps étant immunospécifique pour le complexe CD4-gp120.

29. Anticorps suivant la revendication 27, ledit anticorps ayant une immunospécificité pour ledit complexe CD4-gp120 supérieure à celle pour gp120 ou CD4 seul.

30. Anticorps suivant l'une quelconque des revendications 27 à 29, destiné à être utilisé en médecine.

31. Utilisation d'un anticorps suivant l'une quelconque des revendications 27 à 29, pour la préparation d'un médicament destiné au traitement ou à la prévention de l'infection par le VIH.

32. Utilisation d'un anticorps suivant l'une quelconque des revendications 27 à 29, pour la préparation d'un médicament destiné au traitement ou à la prévention de l'inflammation.

33. Lignée de cellules d'hybridome ayant les caractéristiques d'identification de la lignée cellulaire déposée dans la Advanced Biotechnology Center Inter Lab Cell Line Collection (CBA ICLB), ledit hybridome produisant DB-81.

34. Molécule d'anticorps produite par l'hybridome de la revendication 33.

35. Anticorps qui se lie spécifiquement au même idiotype que l'anticorps produit par l'hybridome de la revendication 33.

36. Procédé pour engendrer une cellule fixée conforme à l'invention, comprenant
(i) l'expression de la protéine d'enveloppe du VIH codée par *gp160* sur la surface de cellules vivantes ;
(ii) la complexation de la protéine d'enveloppe de (i) avec un récepteur CD4 ; et
(iii) la fixation des cellules revêtues de CD4.

37. Procédé suivant la revendication 36, dans lequel ladite protéine d'enveloppe du VIH est exprimée sur la surface de la cellule vivante par transduction de la cellule avec un vecteur codant pour la protéine d'enveloppe du VIH.

38. Procédé suivant la revendication 36 ou 37, dans lequel le récepteur CD4 est exprimé par la cellule vivante.

39. Procédé suivant la revendication 38, dans lequel la cellule vivante exprime ladite protéine d'enveloppe du VIH et ledit récepteur CD4 à partir d'un vecteur unique.

40. Composition pharmaceutique comprenant une cellule fixée suivant l'une quelconque des revendications 18 à 22, et un diluant ou excipient pharmaceutiquement acceptable.

41. Composition pharmaceutique comprenant une population de cellules fixées suivant l'une quelconque des revendications 18 à 22, plus d'un sérotype d'enveloppe du VIH étant exprimé dans la population, et un diluant ou excipient pharmaceutiquement acceptable.
